# EUROPEAN PATENT SPECIFICATION

(11) **EP 3 302 607 B1**
(45) Date of publication and mention of the grant of the patent: **02.06.2021**
(21) Application number: 16724530.7
(22) Date of filing: 29.04.2016
(51) Int. Cl.: A61M 1/16, B01J 20/06, B01J 20/34, B01J 20/02, B01J 20/28

(54) **MANAGEMENT OF RECHARGER EFFLUENT PH**
VERWALTUNG DES PH-WERTES VON AUFLADERABFLUSS
GESTION DU PH D'EFFLUENT DE RECHARGEUR

(30) Priority: 26.05.2015 US 201514722119; 26.05.2015 US 201514722068
(43) Date of publication of application: 11.04.2018
(73) Proprietor: Medtronic, Inc., Minneapolis, MN 55342 (US)
(72) Inventor: MENON, Kanjimpuredathil, Muralikrishna, Bangalore Karnataka 560037 (IN); JEYACHANDRAN, Ramkumar, Bangalore Karnataka 560066 (IN); DUTTA, Sukalyan, Bangalore 560067 (IN); HOBOT, Christopher, M., Rogers, MN 55374 (US); LURA, David, B., Maple Grove, MN 55311 (US)
(74) Representative: Maschio, Antonio
(86) International application number: PCT/US2016/030312
(87) International publication number: WO 2016/191040

(56) References cited:
- WO-A1-2015/060914
- WO-A1-2015/199765
- WO-A1-2015/199863
- WO-A1-2015/199864
- US-A1- 2015 251 161
- US-A1- 2015 251 162

## Description

### FIELD OF THE INVENTION

The invention relates to sorbent rechargers and methods for recharging zirconium phosphate and/or zirconium oxide used in sorbent dialysis. The described systems and methods include rechargers, flow paths, related components, and control logic for simultaneously or independently recharging reusable modules containing zirconium phosphate or zirconium oxide and managing effluent pH to allow disposal of fluid without additional treatment.

### BACKGROUND

Zirconium phosphate and zirconium oxide are used in sorbent dialysis to remove waste and unwanted solutes from spent dialysate. Generally, zirconium phosphate removes ammonium, potassium, calcium, and magnesium ions from dialysate while the zirconium oxide removes anions such as phosphate or fluoride ions. Both materials are usually packaged together in a cartridge of some type or packed in separate cartridges. Usually, sorbent cartridges are discarded and replaced after use. The discarded sorbent cartridges are broken down and the individual materials separated from each other. Because zirconium phosphate and zirconium oxide are expensive and rechargeable, sorbent re-processers treat the recovered zirconium phosphate and zirconium oxide with chemical solutions. Safe disposal of the chemical waste from solutions used to recharge the materials requires additional steps such as neutralizing the recharging solutions. Conventional methods drive up costs and infrastructure requirements, and increase complexity and waste. US2015/0251162 and US2015/0251161 describe methods and apparatuses for recharging sorbent cartridges containing zirconium phosphate or zirconium oxide.

Hence, there is a need for systems and methods that can recharge sorbent materials without the need to separately treat and dispose of the chemical solutions used. There is also a need for a system that can take advantage of solutions necessary to recharge both zirconium oxide and zirconium phosphate to allow for automatic neutralization of the recharging solutions, thus allowing safe disposal without additional treatment. The need extends to coordinating recharging process for both zirconium oxide and zirconium phosphate when only one cartridge is being recharged.

### SUMMARY OF THE INVENTION

The first aspect of the invention is drawn to a sorbent recharger as defined in claim 1. The sorbent recharger comprises at least a first receiving compartment for a first sorbent module; wherein the first receiving compartment has a first sorbent module inlet and a first sorbent module outlet; a first inlet line fluidly connected to the first sorbent module inlet; a first effluent line fluidly connected to the first sorbent module outlet; an disinfectant source, a base source, a water source, and a brine source; wherein at least one of the disinfectant source, base source, water source, and brine source is fluidly connected to the first inlet line; and wherein at least one of the disinfectant source, base source, water source and brine source is fluidly connected to the first effluent line at a junction; and a static mixer at or downstream of the junction,
wherein the water source, disinfectant source, and brine source are fluidly connected to the first inlet line; and wherein at least the water source and base source are fluidly connected to the first effluent line; or
wherein the water source, disinfectant source, and base source are fluidly connected to the first inlet line; and wherein at least the water source and brine source are fluidly connected to the first effluent line.

In any embodiment, the sorbent recharger can include a second receiving compartment for a second reusable sorbent module; the second receiving compartment having a second sorbent module inlet and a second sorbent module outlet; second inlet line fluidly connected to the second sorbent module inlet; a second effluent line fluidly connected to the second sorbent module outlet; and at least one of the disinfectant source, base source, water source, and brine source can be fluidly connected to the second inlet line; and wherein the second effluent line is fluidly connected to the junction.

In any embodiment, the sorbent recharger can have a conductivity sensor positioned in the first effluent line.

In any embodiment, the sorbent recharger can have a conductivity sensor positioned in each of the first effluent line and second effluent line.

In any embodiment, the sorbent recharger can have a first pump in the first inlet line for pumping fluid from the disinfectant source, base source, water source, and/or brine source to the first sorbent module inlet; and a second pump for pumping fluid from the disinfectant source, base source, water source, and/or brine source to the junction.

In any embodiment, the sorbent recharger can have a first pump in the first inlet line for pumping fluid from the disinfectant source, base source, water source, and/or brine source to the first sorbent module inlet; a second pump in the second inlet line for pumping fluid from the disinfectant source, base source, water source, and/or brine source to the second sorbent module inlet; and a controller for controlling the first pump and second pump.

In any embodiment, the controller can be configured to control the first pump to pump fluid from the brine source to the first sorbent module inlet; and to control the second pump to pump fluid from the base source to the second sorbent module inlet concurrently.

In any embodiment, the sorbent recharger can have a least one module bypass line, wherein the module bypass line fluidly connects either the first inlet line to the first effluent line; or the second inlet line to the second effluent line.

In any embodiment, the first sorbent module inlet can be fluidly connectable to the first sorbent module outlet and/or the second sorbent module inlet can be fluidly connectable to the second sorbent module outlet.

The invention is drawn further to a method defined in claim 8, including the steps of pumping a brine solution through a sorbent recharger according to claim 1, the first sorbent module containing zirconium phosphate; and then pumping a resulting solution to a static mixer or common reservoir; and concurrently pumping a base solution to the static mixer.

In any embodiment, the step of pumping the base solution to the static mixer can include the step of pumping the base solution through a second reusable sorbent module, the second reusable sorbent module containing zirconium oxide.

In any embodiment, the step of pumping the base solution to the static mixer can include the step of pumping the base solution through a module bypass line.

In any embodiment, the method can include pumping fluid from the static mixer to a drain.

In any embodiment, the method can include the step of sensing conductivity or pH in an effluent of the module containing zirconium phosphate; and the step of concurrently pumping a base solution to the static mixer can be carried out when the conductivity sensor senses an increase in conductivity, the pH sensor sensing a decrease in pH, or combinations thereof, in the effluent of the module containing zirconium phosphate.

In any embodiment, the method can include the step of determining a pH in the fluid downstream of the static mixer with a sensor; and pumping the fluid from the static mixer to a drain when the pH of the fluid is between 5 and 9.

The invention is drawn further to a method according to claim 10, including the steps of pumping a base solution through a sorbent recharger according to claim 1, the first sorbent module containing zirconium oxide; and then pumping a resulting solution to a static mixer or common reservoir; and concurrently pumping a brine solution to the static mixer.

In any embodiment, the step of pumping the brine solution to the static mixer can include the step of pumping the brine solution through a second reusable sorbent module, the second reusable sorbent module containing zirconium phosphate.

In any embodiment, the step of pumping the brine solution to the static mixer can include the step of pumping the brine solution through a module bypass line.

In any embodiment, the method can include the step of sensing a conductivity or pH in an effluent of the module containing zirconium oxide; and the step of concurrently pumping a base solution to the static mixer can be carried out when the conductivity sensor senses an increase in conductivity, the pH sensor sensing an increase in pH, or combinations thereof, in the effluent of the module containing zirconium oxide.

### BRIEF DESCRIPTION OF THE DRAWINGS

FIG. 1 shows a timeline for concurrent recharging of zirconium oxide and zirconium phosphate.
FIG. 2 shows a timeline for independent recharging of zirconium phosphate.
FIG. 3 shows a timeline for independent recharging of zirconium oxide.
FIG. 4 is a flow chart illustrating the steps for in-line neutralization of the recharger effluent.
FIG. 5A shows a recharging flow path for recharging zirconium phosphate and zirconium oxide.
FIG. 5B shows a recharging flow path for recharging zirconium phosphate and is an exploded left side of FIG. 5A.
FIG. 5C shows a recharging flow path for recharging zirconium oxide and is an exploded right side of FIG. 5A.
FIG. 6A shows a recharging flow path for recharging zirconium phosphate and zirconium oxide with in-line mixing of recharging solutions.
FIG. 6B shows a recharging flow path for recharging zirconium phosphate with in-line mixing of recharging solutions and is an exploded right side of FIG. 6A.
FIG. 6C shows a recharging flow path for recharging zirconium oxide with in-line mixing of recharging solutions and is an exploded left side of FIG. 6A.
FIG. 7 shows a recharger for recharging zirconium phosphate and zirconium oxide modules.
FIG. 8 shows a recharger fluidly connected to external fluid sources.
FIG. 9 shows multiple rechargers fluidly connected to a single set of fluid sources.
FIG. 10 shows material layers in a module sorbent cartridge including reusable modules.
FIG. 11 shows multiple sorbent modules connected together to form a sorbent cartridge.

### DETAILED DESCRIPTION OF THE INVENTION

Unless defined otherwise, all technical and scientific terms used herein generally have the same meaning as commonly understood by one of ordinary skill in the relevant art.

The articles "a" and "an" are used herein to refer to one or to more than one (*i.e.,* to at least one) of the grammatical object of the article. By way of example, "an element" means one element or more than one element.

A "base solution" is any aqueous solution with a pH of greater than 7.

A "base source" is a fluid or concentrate source from which a basic solution can be obtained.

A "brine solution" refers to any solution comprising acids, bases, and/or salts.

A "brine source" is a fluid or concentrate source from which a brine solution can be obtained. As used herein, a brine solution can refer to any solution comprising acids, bases and/or salts.

A "common reservoir" can be a container for collecting fluid of any type from one or more fluid sources including fluid lines or other reservoirs. The "common reservoir" can for example, store used or waste fluids.

The term "comprising" includes, but is not limited to, whatever follows the word "comprising." Use of the term indicates the listed elements are required or mandatory but that other elements are optional and may be present.

The term "concurrently" refers to two processes or events taking place at the same time.

The term "conductivity" refers to the inverse of the resistance of a material.

A "conductivity sensor" is a sensor configured to measure the conductivity of a fluid.

The term "consisting of' includes and is limited to whatever follows the phrase "consisting of." The phrase indicates the limited elements are required or mandatory and that no other elements may be present.

The term "consisting essentially of' includes whatever follows the term "consisting essentially of' and additional elements, structures, acts or features that do not affect the basic operation of the apparatus, structure or method described.

The terms "contain," "containing," or "contained" as used herein means to keep a material within a specific place. "Contain" can refer to materials placed within a component, absorbed onto a component, bound to a component, or any other method of keeping the material in a specific place.

The term "control" or "configured to control" refers to the ability of one component to direct the actions of a second component.

A "controller" is any device which monitors and affects the operational conditions of a system. The operational conditions are typically referred to as output variables of the system wherein the output variables can be affected by adjusting certain input variables.

The terms "determining" and "determine" refer to ascertaining a particular state of a system or variable(s).

A "disinfectant source" is a fluid or concentrate source from which a disinfectant solution can be obtained. The disinfectant solution can be an acidic solution, such as a peracetic acid solution, or any other solution capable of disinfecting reusable sorbent modules.

The term "downstream" refers to a position of a first component in a flow path relative to a second component wherein fluid will pass by the second component prior to the first component during normal operation. The first component can be said to be "downstream" of the second component, while the second component is "upstream" of the first component.

A "drain" is a fluid line through which fluids may be disposed.

The term "effluent" refers to fluid exiting a container, compartment, or cartridge.

An "effluent line" is a fluid passageway, tube, or path of any kind into which fluid exiting a container, module, or component will flow.

A "fluid" is a liquid substance optionally having a combination of gas and liquid phases in the fluid. Notably, a liquid, as used herein, can therefore also have a mixture of gas and liquid phases of matter.

The term "fluidly connectable," "fluidly connect," "for fluid connection," and the like, refer to the ability of providing for the passage of fluid, gas, or a combination thereof, from one point to another point. The two points can be within or between any one or more of compartments, modules, systems, components, and rechargers, all of any type. The connection can optionally be disconnected and then reconnected.

A "fluid connector," "fluid connection," and the like describe a connection between two components wherein fluid, gas, or combination thereof, can flow from one component, through a connector or a component for connection, to another component. The connector provides for a fluid connection in its broadest sense and can include any type of tubing, fluid or gas passageway, or conduit between any one or more components of the invention. The connection can optionally be disconnected and then reconnected.

The term "fluid line mixing" refers to mixing fluids at a location or junction wherein flow at the location of junction can, in part, mix one or more fluids.

A "fluid source" is a source from which a fluid or concentrate may be obtained.

An "inlet line" is a fluid line through which fluid entering a container, module, or component will flow.

A "junction" is a location where at least two fluid lines are connected to each other, with or without a valve.

The term "mixing" generally refers to causing one or more fluids from any source to combine together. For example, "mixing" can include laminar or turbulent flow at a location in a fluid line or a junction. Another example of "mixing" can include receiving one or more fluids in a component configured to receive fluids from one or multiple sources and to mix the fluids together in the component. Additionally, mixing can refer to the dissolution of a solid or solids with a fluid, wherein the solid or solids is dissolved in the fluid.

A "module bypass line" refers to a fluid line that provides for movement of fluid between two points without passing through a module.

The term "pH sensor" refers to a device for measuring the pH or hydrogen ion concentration of a fluid.

The term "positioned" or "position" refers to a physical location of a component or structure.

The term "pressure sensor" refers to a device for measuring the pressure of a gas or liquid in a vessel, container, or fluid line.

The term "pump" refers to any device that causes the movement of fluids, gases, or combinations thereof, by applying suction or pressure.

The terms "pumping," "pumped," or to "pump" refer moving a fluid, gas, or combination thereof, with a pump.

A "receiving compartment" is a space within a recharger into which a sorbent module to be recharged is placed.

A "sorbent recharger" is an apparatus designed to recharge at least one sorbent material.

"Recharging" refers to treating a sorbent material to restore the functional capacity of the sorbent material to put the sorbent material back into a condition for reuse or use in a new dialysis session. Sometimes, the total mass, weight and/or amount of "rechargeable" sorbent materials remain the same. Sometimes, the total mass, weight and/or amount of "rechargeable" sorbent materials change. Without being limited to any one theory of invention, the recharging process may involve exchanging ions bound to the sorbent material with different ions, which sometimes may increase or decrease the total mass of the system. However, the total the sorbent material will sometimes be unchanged by the recharging process. Upon a sorbent material undergoing "recharging," the sorbent material can then be said to be "recharged." Recharging of rechargeable sorbent materials is not the same as replenishing of a sorbent material such as urease. Notably, urease is not "recharged," but can be replenished, as defined herein.

A "recharging flow path" is a path through which fluid can travel while recharging sorbent material in a reusable sorbent module.

The term "resulting solution" refers to a solution that is the consequence from one or more prior steps.

The terms "sensing," "sensed" or to "sense" refer to determining one or more parameter or variable.

A "sensor" is a component capable of determining or sensing the states of one or more variables in a system.

A "sorbent cartridge module" or "sorbent module" means a discreet component of a sorbent cartridge. Multiple sorbent cartridge modules can be fitted together to form a sorbent cartridge of two, three, or more sorbent cartridge modules. In some embodiments, a single sorbent cartridge module can contain all of the necessary materials for dialysis. In such cases, the sorbent cartridge module can be a "sorbent cartridge."

A "sorbent module inlet" is a connector through which a fluid, slurry, or aqueous solution can enter a sorbent module.

A "sorbent module outlet" is a connector through which a fluid, slurry, or aqueous solution can exit a sorbent module.

A "static mixer" is a component configured to receive fluids from one or multiple sources and to mix the fluids together. The static mixer may include components that agitate the fluids to further mixing.

The term "upstream" refers to a position of a first component in a flow path relative to a second component wherein fluid will pass by the first component prior to the second component during normal operation. The first component can be said to be "upstream" of the second component, while the second component is "downstream" of the first component.

A "water source" is a fluid source from which water can be obtained.

### Recharger Effluent pH Management

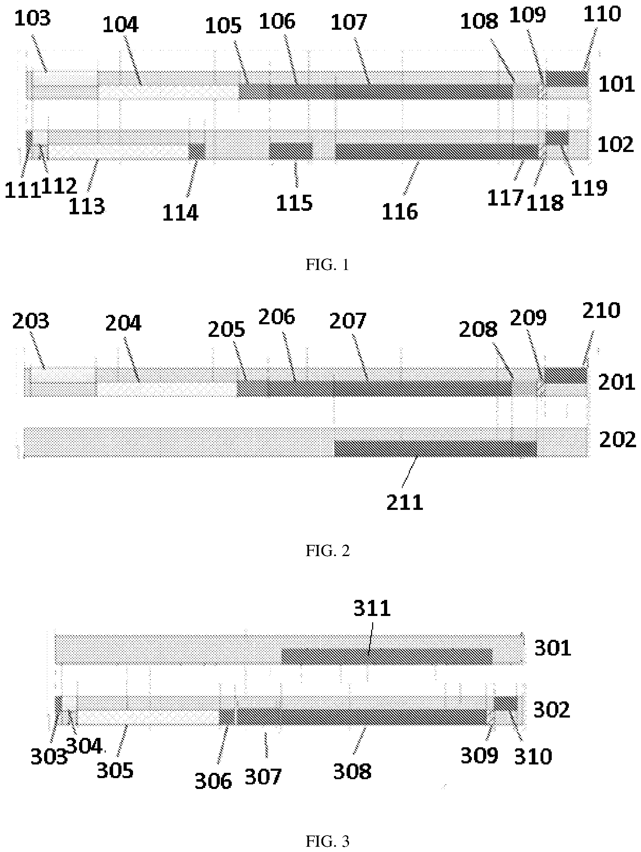

FIG. 1 illustrates a non-limiting timeline that uses acidic effluent from the zirconium phosphate recharging process to neutralize the basic effluent from the zirconium oxide recharging process and vice versa. Timeline **101** shows recharging zirconium phosphate and timeline **102** shows recharging zirconium oxide. As illustrated in timeline **101,** the zirconium phosphate recharging process can begin by introducing a disinfectant, such as peracetic acid, into the zirconium phosphate module, shown as step **103.** The disinfectant can be any disinfectant capable of disinfecting the sorbent modules, including citric acid, bleach, or any other disinfectant known in the art. The time necessary to fill the zirconium phosphate module with the disinfectant can depend on the flow rate of the disinfectant solution and the volume of the zirconium phosphate module. The disinfectant can be delivered to the zirconium phosphate module in step **103** at a flow rate of between 200 and 500 mL/min, which can fill a zirconium phosphate module in a time of between 5-10 minutes. After filling the zirconium phosphate with the disinfectant solution, the disinfectant solution can be held in the zirconium phosphate module to ensure disinfecting of the zirconium phosphate module in step **104.** In any embodiment, the disinfectant can be held in the zirconium phosphate module for any length of time sufficient to disinfect the zirconium phosphate module, including between 5 and 20 minutes. The temperature of the disinfectant can be determined with a temperature sensor, and a holding time adjusted as necessary. For example, if the disinfectant temperature is 22°C, the hold time can be 5 minutes. The disinfectant can also be heated to minimize the hold time by heating the disinfectant to room temperature. During the hold time, the disinfectant flow can be stopped or reduced to a low flow condition, such as 5 to 75 ml/min. Sequestering the disinfectant, such as peracetic acid, in the module can build up pressure in the module, requiring periodic venting. To maintain the volume after venting, during which some fluid may leak, the disinfectant can be pumped into the module at a low flow rate during the venting process. Alternatively, the disinfectant flow rate can be set to between 5 and 75 ml/min during the hold time to prevent pressure buildup while maintaining fluid volume in the modules. The disinfectant solution can then be flushed from the zirconium phosphate module in step **105** by pumping water through the zirconium phosphate module. The water can flow through the zirconium phosphate module at a specified rate. A higher flow rate of the water in step **105** will cause a quicker flush time. The water can be pumped through the zirconium phosphate module at a rate of between 100 and 500 mL/min. Depending on the size of the zirconium phosphate module, the zirconium phosphate module can be flushed in one non-limiting example for about 5-10 minutes. Longer or shorter flushing times can be used depending on the need. As described, the system can utilize one or more sensors, such as pH sensors or conductivity sensors in zirconium phosphate effluent lines to sense the pH or conductivity of the effluent and to determine if disinfectant has been fully flushed in step **105.** After flushing disinfectant from the zirconium phosphate module in step **105,** a brine solution can be pumped through the zirconium phosphate module to recharge the zirconium phosphate module in step **106.** The brine solution can be pumped through the zirconium phosphate module in step **106** at any rate. One of skill in the art will understand that a higher flow rate of brine solution may decrease the time necessary to recharge the zirconium phosphate, but may also decrease the efficiency of the process, resulting in additional brine. Conductivity or pH sensors can determine the pH or conductivity of the effluent and to determine if the zirconium phosphate module has been fully filled with brine. The brine flow rate can be set to any flow rate, including between 150 and 250 mL/min. Depending on the size of the zirconium phosphate module, between 5 and 10 minutes may be needed for brine to reach the sensors in the zirconium phosphate effluent line. Once brine has reached sensors in the effluent line, the brine can flow through the zirconium phosphate module in step **107** until recharging is complete.

Recharging time can vary based on the flow rate of the brine solution, the concentration of the brine solution, and the temperature of the brine solution. For example, the brine solution can be heated during the recharging process between 65°C and 95°C. Recharging of zirconium phosphate can be more efficient at elevated temperatures. Conductivity sensors can determine if step **108** has been completed by sensing the conductivity of the fluid in the zirconium phosphate effluent line. If the conductivity of the effluent matches the conductivity of the brine, then no additional ions from the brine are being exchanged onto the zirconium phosphate, and recharging is complete. For example, steps **108, 109,** and **110** represent brine solution being flushed from the zirconium phosphate module with water. Flushing can continue through step **110** until the conductivity sensors in the zirconium phosphate effluent line determine no additional brine is being removed from the zirconium phosphate module.

As depicted in timeline **102** of FIG. 1, zirconium oxide can be recharged concurrently or independently of zirconium phosphate. In step **111,** zirconium oxide recharging begins by rinsing the zirconium oxide module with water. The water rinse can flush leftover dialysate bicarbonate or any sodium hydroxide from the flow loop, which may react violently with acid necessary for disinfection. After flushing the zirconium oxide module with water in step **111,** disinfectant solution can be delivered to disinfect the module in step **112.** The time necessary to fill the zirconium oxide module with disinfectant depends on the size of the zirconium oxide module and the flow rate of the disinfectant. Because less zirconium oxide is needed for dialysis than zirconium phosphate, the zirconium oxide module may be smaller than the zirconium phosphate module, and therefore fill faster in step **112** as compared to the zirconium phosphate module in step **103.** Upon filling, the disinfectant can be sequestered in the zirconium oxide module to allow for disinfection in step **113.** The disinfectant can be held in the zirconium oxide module for any length of time, including between 5 and 20 minutes. The temperature of the disinfectant can be determined with a temperature sensor, and a hold time adjusted as necessary. For example, if the disinfectant temperature is 22°C, the hold time can be 5 minutes. The disinfectant can also be heated to minimize the necessary hold time. Upon disinfection, the disinfectant can be flushed from the zirconium oxide module in step **114.**

In step **115** the base solution flows through the zirconium oxide module to recharge the zirconium oxide. Step **115** continues until a basic solution is detected in the zirconium oxide effluent line. During simultaneous recharging, the basic effluent from the zirconium oxide recharging flow path neutralizes the acidic effluent from the zirconium phosphate recharging flow path. Once a basic effluent is detected in step **115,** the zirconium oxide recharging process can be halted until the acid brine is detected in the effluent of the zirconium phosphate module in step **106,** which may occur later due to size differences of the zirconium phosphate and zirconium oxide modules. After the acidic effluent is detected in the zirconium phosphate module, shown as step **106,** the base can continue to flow through the zirconium oxide module in step **116** concurrently with the brine solution flowing through the zirconium phosphate module. The flow rate of the base solution in step **116** can be any suitable rate. For example, the flow rate of the base solution can be between 30 and 150 mL/min. To ensure neutralization, the flow rate of the base in step **116** can depend on the flow rate of the brine in step **107.** As described, the base and effluent are each brought to a point equidistant to a junction between the zirconium phosphate and zirconium oxide effluent lines. Based on the conductivity of each effluent, the pumping is restarted at a ratio of speed that is needed for neutralization. The ratio could be 1:1 or any other ratio. Although described as using a conductivity sensor, the system can alternatively use a pH sensor or a combination of pH and conductivity sensors. A neutralization ratio can be calculated based on the relative pH, buffer capacity, and concentration of the zirconium phosphate effluent and zirconium oxide effluent. For example, a neutralization ratio of 1.5:1 means that 1.5 liters of the zirconium phosphate effluent will be required to fully neutralize one liter of zirconium oxide effluent. The flow rate of the base in step **116** can be set to half the flow rate of the brine solution, allowing full neutralization of both solutions. For example, the flow rate of the base in step **116** can be between 75 and 125 mL/min if the neutralization ratio is 1.5:1 and the brine flow rate is between 150 and 250 mL/min.

After the brine solution is detected in the effluent of the zirconium phosphate and the flushing of the brine begins in step **108,** the base solution can pass through the zirconium oxide module, shown as step **117** until the brine is mostly or fully flushed from the zirconium phosphate module, shown as step **109.** At this point, the base solution can be flushed from the zirconium oxide module, shown as step **118.** After confirming that the base has been flushed from the zirconium oxide module, flushing is completed in step **119.**

One of skill in the art will understand that the times and flow rates represented by FIG. 1 can be altered within the scope of the invention. Higher flow rates can cause faster recharging of the modules. Times can be decreased by using more concentrated solutions, but may decrease efficiency. Specified concentrations, flow rates, and times can be set per the needs of the user, taking into account the cost of chemicals and need for fast recharging. The times and flow rates shown in zirconium oxide recharging timeline **102** can be altered to reduce idle time. For example, the flow rate of the base solution in step **115** can be slowed down to reduce the time gap between steps **115** and **116.** If a common reservoir is used for the zirconium phosphate and zirconium oxide recharging flow paths either inside or outside of the recharger, the times and flow rates shown in FIG. 1 can be adjusted. Synchronizing the zirconium phosphate timeline **101** with the zirconium oxide timeline **102** is unnecessary because the combined effluent is not directly disposed in a drain.

FIG. 2 illustrates a non-limiting example of a timeline that can be used for independent recharging of zirconium phosphate using the dual recharging flow path described herein. Timeline **201** shows recharging zirconium phosphate and timeline **202** shows the process for in-line neutralization of the zirconium phosphate effluent without recharging a zirconium oxide module. As illustrated in timeline **201,** the zirconium phosphate recharging process can begin by introducing a disinfectant, such as peracetic acid, into the zirconium phosphate module, shown as step **203.** After filling the zirconium oxide with the disinfectant solution, the disinfectant solution can be sequestered in the zirconium oxide module to ensure disinfecting of the zirconium phosphate module in step **204.** The disinfectant solution can then be flushed from the zirconium phosphate module in step **205** by pumping water through the zirconium phosphate module at a specified rate. As described, the system can utilize one or more sensors, such as pH sensors or conductivity sensors in the zirconium phosphate effluent lines to determine if disinfectant is fully flushed in step **205.** After flushing the disinfectant from the zirconium phosphate module in step **205,** brine solution can be pumped through the zirconium phosphate module to recharge the zirconium phosphate module starting in step **206.** Once brine has reached the sensors in the effluent line, the brine can flow through the zirconium phosphate module in step **207** until recharging is complete. Concurrently, a base solution can be pumped through the zirconium oxide recharging flow path in step **211** to neutralize the brine solution.

Conductivity sensors can determine if step **208** has been completed by sensing the conductivity of the fluid in the zirconium phosphate effluent line. If the conductivity of the effluent matches the conductivity of the brine, then no additional ions from the brine are being exchanged onto the zirconium phosphate, and recharging is complete. For example, steps **208, 209,** and **210** represent brine solution being flushed from the zirconium phosphate module with water. Flushing can continue through step **210** until the conductivity sensors in the zirconium phosphate effluent line determine no additional brine is being removed from the zirconium phosphate module. Once the conductivity sensors determine that the pH of the zirconium phosphate effluent is safe for disposal without additional treatment, the base solution in the zirconium oxide recharging flow path is stopped. The fluid flow rates and concentrations used in the process illustrated in FIG. 2 can be the same as the fluid flow rates and concentrations described with reference to FIG. 1.

FIG. 3 shows a timeline for independently recharging zirconium oxide. Timeline **302** shows the recharging of zirconium oxide and timeline **301** shows using the zirconium phosphate recharging flow path for in-line neutralization of the zirconium oxide effluent. In step **303,** zirconium oxide recharging begins by rinsing the zirconium oxide module with water to flush leftover dialysate bicarbonate, which may react violently with acid necessary for disinfection. After flushing the zirconium oxide module with water in step **303,** disinfectant solution can be delivered to disinfect the module in step **304.** Upon filling, the disinfectant can be sequestered in the zirconium oxide module to allow for disinfection in step **305.** Upon disinfection, the disinfectant can be flushed from the zirconium oxide module in step **306.**

In step **307** the base solution flows through the zirconium oxide module to recharge the zirconium oxide. Step **307** continues until a basic solution is detected in the zirconium oxide effluent line. Once the basic solution is detected in the zirconium oxide effluent line, brine is concurrently pumped through the zirconium phosphate recharging flow path for in-line neutralization of the basic zirconium oxide effluent in step **311.** The base solution continues to flow through the zirconium oxide module until recharging is complete in step **308.** After recharging the zirconium oxide in step **308,** the basic solution can be flushed in steps **309** and **310.** Conductivity sensors in the zirconium oxide effluent line determine when the basic solution is fully flushed, at which point the brine solution in step **311** can be stopped. The process illustrated in FIG. 3 can use the same flow rates and concentrations as described with respect to FIG. 1.

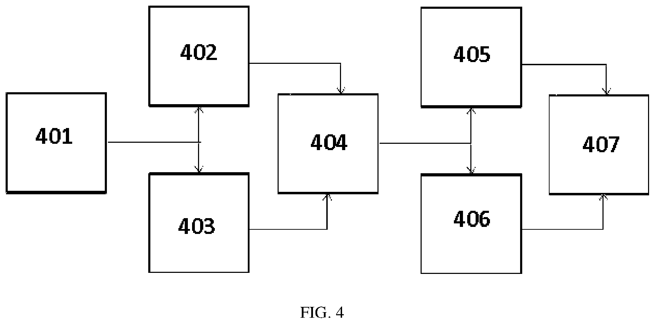

FIG. 4 provides a flow chart illustrating the steps for in-line neutralization of the zirconium phosphate recharging effluent and zirconium oxide recharging effluent. In step **401,** the zirconium oxide module can be flushed with water, and each of the zirconium oxide and zirconium phosphate modules can be filled with disinfectant for disinfection, followed by flushing of the disinfectant solution. After disinfection and flushing, the zirconium phosphate module can be filled with brine in step **402** and the zirconium oxide module can be filled with a base in step **403.** The conductivity or pH of the effluent of the zirconium oxide module and zirconium phosphate module can be sensed by a conductivity or pH sensor in step **404.** Steps **402** and **403** are continued until the system senses both brine in the zirconium phosphate effluent and base in the zirconium phosphate effluent in step **404.** If the system detects only base in the zirconium oxide effluent but not brine in the zirconium phosphate effluent, step **403** can be halted until the brine is detected in the zirconium phosphate effluent. After detecting both brine in the zirconium phosphate effluent and base in the zirconium oxide effluent in step **404,** the recharging process can continue with pumping brine through the zirconium phosphate module in step **405** and pumping base through the zirconium oxide module in step **406.** The conductivities of the zirconium phosphate and zirconium oxide effluents can be sensed in step **407,** and steps **405** and **406** continued until no change is detected in the conductivity of each of the effluents in step **407.** After the system determines there is no change in the effluent conductivity of each of the modules in step **407,** recharging is complete and the modules can be rinsed with water and reused. At any point during step **407** a pH sensor or conductivity sensor can sense the combined effluent of the zirconium phosphate and zirconium oxide modules to ensure that the combined effluent has a pH safe for disposal. In any embodiment, the system can ensure that the combined effluent has a pH of between 5-9. If the pH is outside of this range, the system can prevent the disposal of the combined effluent.

In any embodiment, the system can include a controller for controlling the pumps and valves described. The controller can receive data from the conductivity or pH sensors and control the pumps and valves to carry out the processes described with in-line neutralization of the recharging effluent.

The zirconium oxide and zirconium phosphate sorbent modules can be recharged and reused any number of times. Alternatively, the sorbent modules may have a defined useful life, including a maximum number of recharge and reuse cycles. When a sorbent module reaches the end of the sorbent module's useful life, the sorbent module can be recycled or disposed of. A disinfection only cycle can disinfect the sorbent modules for safe disposal and/or recycling at the end of the sorbent module's useful life. In a disinfection only cycle, the disinfectant can be pumped into the sorbent module as described but the other recharge solutions would not be used. After disinfection, and optionally rinsing of the sorbent module, the sorbent module can be disposed or recycled safely.

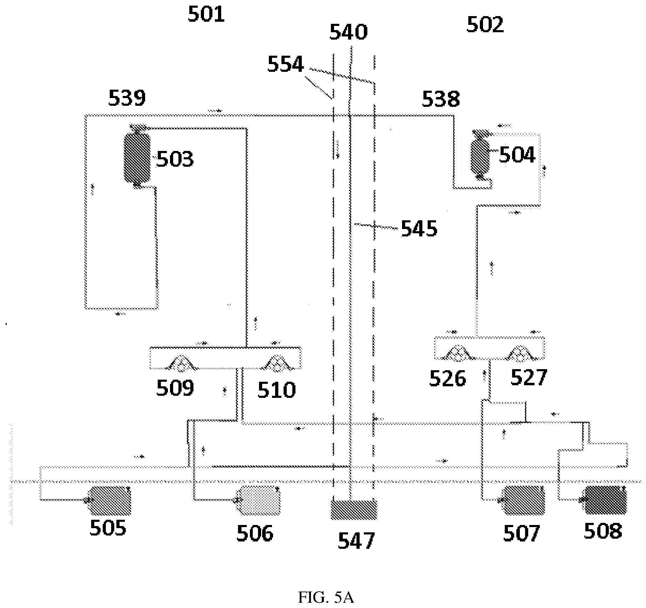

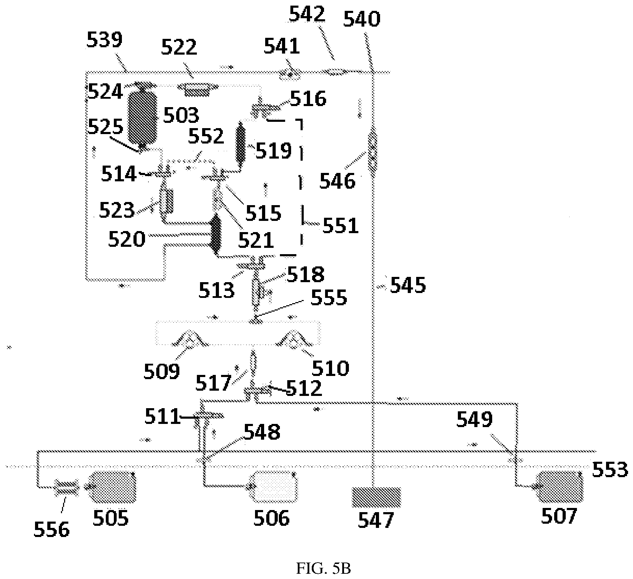

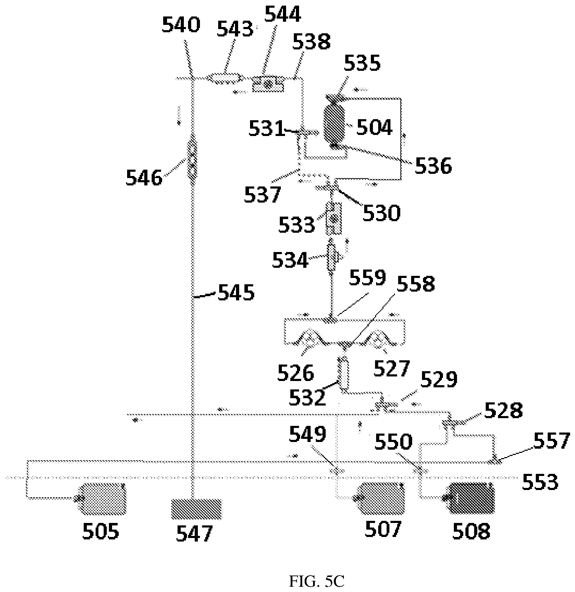

To recharge the sorbent materials, fluids from fluid sources are passed through the sorbent modules. The flow paths can be arranged as shown in FIG.'s 5A-C. FIG. 5A is a generalized view of a recharging flow path, with details shown in FIG.'s 5B and 5C. The recharging flow path can be divided into a zirconium phosphate recharging flow path **501** containing the zirconium phosphate module **503** and a zirconium oxide recharging flow path **502** containing zirconium oxide module **504.** Details of the zirconium phosphate recharging flow path **501 on** the zirconium phosphate side of line **554** are illustrated in FIG. 5B, while details of the zirconium oxide recharging flow path **502** on the zirconium oxide side of line **554** are illustrated in FIG. 5C. Although a dual cartridge recharger system is shown, single, two or more multiple cartridge recharger systems are envisioned. Any one of the recharger cartridge systems can be linked together to share resources for recharging the sorbent cartridge and can be adapted for large scale use. Similarly, the linked rechargers can be scaled down as demand for recharging decreases. The modular recharging set-up having more or less rechargers based on demand can be advantageously used where required.

In FIG. 5A, a zirconium phosphate recharging flow path **501** and a zirconium oxide recharging flow path **502** have a water source **505,** a brine source **506,** a disinfectant source **507,** and a base source **508.** The brine source **506,** disinfectant source **507,** and/or base source **508** can be a column containing a dry bed of the brine, acid, and/or base components. Alternatively, a powdered source of the brine, acid, and/or base components can be used. The dry bed or powdered source can be dissolved with an aqueous solution. A static mixer (not shown) can mix the single line coming through the column prior to entering the zirconium phosphate module **503** or zirconium oxide module **504.** Recharging the zirconium phosphate in a zirconium phosphate module **503** requires water, brine, and disinfectant. The water source **505,** the brine source **506,** and the disinfectant source **507** are fluidly connected to the zirconium phosphate recharging flow path **501.** Similarly, recharging zirconium oxide module **504** in zirconium oxide recharging flow path **502** requires water, base, and disinfectant. The water source **505,** the disinfectant source **507,** and the base source **508** are fluidly connected to the zirconium oxide recharging flow path **502.** The zirconium phosphate recharging flow path **501** and zirconium oxide recharging flow path **502** can be operated simultaneously or independently. Disinfectant source **507** can contain any type of disinfectant compatible with zirconium phosphate and zirconium oxide that is capable of disinfecting the reusable sorbent modules. In any embodiment, the disinfectant source **507** can contain peracetic acid. In any embodiment, the peracetic acid can be a solution of between 0.5 % and 2% peracetic acid in water. The brine source **506** can have an acid, a base, and a sodium salt.

During zirconium phosphate recharging, potassium, calcium, magnesium, and ammonium ions bound to the zirconium phosphate must be replaced by hydrogen and sodium ions. The final ratio of hydrogen to sodium ions on the recharged zirconium phosphate can be determined by the pH, buffer capacity, and sodium concentration of the brine solution used in the recharging process. The brine source **506** can be a mixture of sodium chloride, sodium acetate, and acetic acid. In one non-limiting brine solution, the sodium chloride concentration can be between 2.5 M and 4.9 M, the sodium acetate concentration can be between 0.3 M and 1.1 M, and acetic acid concentration can be between 0.2 M and 0.8 M. The water source **505** can contain any type of water, including deionized water. To recharge the zirconium phosphate in the zirconium phosphate module **503,** the disinfectant from disinfectant source **507** can flow to the zirconium phosphate module **503** to disinfect the zirconium phosphate module **503.** Fluid from the disinfectant source **507** can flow to valve **512** in the zirconium phosphate recharging flow path **501.** Zirconium phosphate pumps **509** and **510** provide a driving force to pump the fluid through the zirconium phosphate recharging flow path **501.** Use of two or more separate pumps can reduce wear on the pumps. Correspondingly, smaller pumps can be used. The two or more pumps can provide in-line mixing and intermittent pumping so at any given time, a single pump can pump fluid through the zirconium phosphate recharging flow path **501.** The two pumps can be used simultaneously or independently. The two or more pumps can provide fluid line mixing for one or more separate fluid streams when used simultaneously. The two or more pumps can operate asynchronously but used concurrently. For example, a first pump can operate for a time and a second pump remain off, then the first pump shut off with the second pump turning on. Multiple pumps at various timed pumping stages are envisioned as described herein. One of skill in the art will understand that a single zirconium phosphate pump can also accomplish the described pump functions.

Zirconium phosphate pumps **509** and **510** can pump fluid from disinfectant source **507** through valve **512** and valve **513.** Fluid can be pumped through three-way junction **555** to valve **516** and into zirconium phosphate module **503** through zirconium phosphate sorbent module inlet **524.** The illustrated junctions combine the inlet chemicals or water pumped by the two pumps such that higher flow rates can be achieved. During filling, fluid inside zirconium phosphate module **503** can be forced through zirconium phosphate sorbent module outlet **525** and into zirconium phosphate module effluent line **539.** The disinfectant can be sequestered in the zirconium phosphate module **503** to ensure disinfection. Heater **519** upstream of the zirconium phosphate module **503** can heat the disinfectant because disinfection can become more efficient at elevated temperatures. After disinfection, zirconium phosphate module **503** can be rinsed using water from water source **505.** Zirconium phosphate pumps **509** and **510** can pump water from water source **505** through valves **511** and **512** to valve **513.** The water can then be pumped through valves **515** and **516** through the zirconium phosphate module **503** through zirconium phosphate sorbent module inlet **524,** out zirconium phosphate sorbent module outlet **525** and into zirconium phosphate module effluent line **539.** Water can be pumped through the zirconium phosphate module **503** until all of the disinfectant is removed.

Fluid from brine source **506** can be pumped through the zirconium phosphate module **503** to load the zirconium phosphate module **503** with the proper ratio of sodium and hydrogen ions. Zirconium phosphate pumps **509** and **510** can pump fluid from brine source **506** to valve **511.** The brine can follow the same pathway as the water through zirconium phosphate module **503** and into zirconium phosphate module effluent line **539.** Heater **519** upstream of the zirconium phosphate module **503** can heat brine because recharging can become more efficient at elevated temperatures. Heat exchanger **520** can lessen the load on heater **519.** One or more heat exchangers and one or more heaters can be used. The heat exchanger **520** can be fluidly connected to zirconium phosphate module effluent line **539** and to zirconium phosphate sorbent module inlet **524** upstream of heater **519.** The heated fluid exiting the zirconium phosphate module **503** in zirconium phosphate module effluent line **539** can heat the incoming brine solution in heat exchanger **520.** The heat exchanger **520** can have at least a first chamber and a second chamber. Fluid in the zirconium phosphate inlet lines can pass through the first chamber of the heat exchanger **520,** and fluid in the zirconium phosphate effluent line **539** can pass through the second chamber of the heat exchanger **520.** The increased temperature of the zirconium phosphate effluent in the second chamber can heat the fluid in the zirconium phosphate inlet lines in the first chamber. The zirconium phosphate module **503** can be rinsed again by pumping water through the zirconium phosphate module **503.** A static mixer (not shown) can be positioned upstream of the zirconium phosphate module **503** and mix the solutions prior to entering the zirconium phosphate module **503.**

Various sensors can be used in the zirconium phosphate module recharging flow path **501** to ensure proper concentrations and temperatures as shown in FIG. 5B. For example, conductivity sensor **517** can ensure that the incoming water contains no level of ions that may interfere with the recharging process, and that the brine solution and disinfectant solution are at a desired concentration. Conductivity sensor **517** can also ensure that sufficient rinsing has occurred to remove brine and disinfectant solution. Pressure sensor **518** can monitor pressure in the zirconium phosphate inlet lines to ensure there are no occlusions or leaks and that the inlet pressures are in an acceptable range. Temperature sensor **522** can ensure that the brine solution is at the proper temperature before entering zirconium phosphate module **503** and to control heater **519.** Temperature sensor **523** can be placed in zirconium phosphate module effluent line **539** to monitor the temperature of the effluent which can be controlled by heat exchanger **520** and heater **519.** A flow sensor **521** can monitor the flow rates of the fluids in the zirconium phosphate recharging flow path **501** and control zirconium phosphate pumps **509** and **510.** One of skill in the art will understand that alternative arrangements of sensors can be used in FIG. 5B and that one or more additional sensors can be added. Further, the sensors can be placed at any appropriate position in the zirconium phosphate recharging flow path **501** to determine fluid parameters at various locations throughout the zirconium phosphate recharging flow path **501.**

Zirconium phosphate module bypass line **552** fluidly connects valve **515** to valve **514** in the zirconium phosphate effluent line **539.** Valves **515** and **516** can be controlled to direct fluid through the zirconium phosphate module bypass line **552** and into zirconium phosphate effluent line **539.** The dual flow path aspect of the recharging flow path depicted in FIG. 5A can neutralize the effluent from both the zirconium phosphate module **503** and zirconium oxide module **504** by mixing the acidic effluent from the zirconium phosphate module **503** with the basic effluent from zirconium oxide module **504.** If only zirconium oxide module **504** is being recharged using the flow path of FIG. 5C, the zirconium phosphate module bypass line **552** can be utilized to direct fluid from the brine source **506** to the zirconium phosphate effluent line **539** to neutralize the zirconium oxide effluent without the need to simultaneously recharge a zirconium phosphate module **503.** Alternatively, zirconium phosphate sorbent module inlet **524** can directly connect to zirconium phosphate sorbent module outlet **525.** The zirconium phosphate recharging flow path **501** can include a rinse loop **551** to fluidly connect valve **513** upstream of the heater **519** and heat exchanger **520** to valve **516,** bypassing heater **519** and heat exchanger **520.** The rinse loop **551** can rinse brine solution from the zirconium phosphate module **503.** By bypassing heater **519** and heat exchanger **520** through rinse loop **551,** the zirconium phosphate module **503** can be cooled faster.

To recharge the zirconium oxide module **504,** disinfectant from disinfectant source **507** can be first pumped to the zirconium oxide module **504** to disinfect the zirconium oxide module **504.** Fluid from the disinfectant source **507** can be pumped to valve **529** in the zirconium oxide recharging flow path **502.** Zirconium oxide pumps **526** and **527** can pump fluid through the zirconium oxide recharging flow path **502.** As described, a single zirconium oxide pump is contemplated as an alternative to the dual pump system in FIG. 5. Also, two or more zirconium oxide pumps are contemplated. The two or more zirconium oxide pumps can provide fluid line mixing of one or more separate fluid streams when used simultaneously. The two or more pumps can be asynchronous but used concurrently. For example, a first pump can operate for a time and a second pump remain off, then the first pump shut off with the second pump turning on. Multiple pumps at various timed pumping stages are envisioned as described herein. Zirconium oxide pumps **526** and **527** pump fluid from disinfectant source **507** through valve **529** to valve **530.** The fluid flows to the zirconium oxide module **504** through zirconium oxide sorbent module inlet **535.** During filling, fluid inside zirconium oxide module **504** can flow through zirconium oxide sorbent module outlet **536** and into zirconium oxide module effluent line **538.** The disinfectant can be sequestered in zirconium oxide module **504** to ensure disinfection. The zirconium oxide module **504** can then be flushed with water from water source **505** after disinfection is completed. Zirconium oxide pumps **526** and **527** can pump water from water source **505** through valves **528** and **529** and junction **557** to valve **530.** The fluid passes through junctions **558** and **559** to reach valve **530.** The water can then be pumped to zirconium oxide module **504** through zirconium oxide module inlet **535** and out zirconium oxide sorbent module outlet **536** and into zirconium oxide module effluent line **538.** The zirconium oxide module **504** can be flushed with any volume of water required to ensure that the disinfectant is completely removed.

In FIG. 5C, zirconium oxide pumps **526** and **527** can pump fluid from base source **508** through valve **528** to zirconium oxide module **504.** The base source **508** can contain hydroxide ions to recharge zirconium oxide module **504.** The hydroxide ions can flow through zirconium oxide module **504** and into zirconium oxide module effluent line **538.** The base source **508** can be any suitable basic solution capable of replacing phosphate and other anions bound to the zirconium oxide with hydroxide ions. The hydroxide base can be any suitable base such as sodium hydroxide. One non-limiting example is sodium hydroxide having a concentration between 0.5M and 2.0M. Another non-limiting example is sodium hydroxide having a concentration at 90% or greater than 2% of the concentration of the recharging solution. A final rinse of the zirconium oxide module **504** can be performed by pumping water through the zirconium oxide recharging flow path **502** and zirconium oxide module **504.** Zirconium oxide recharging flow path **502** can also have a zirconium oxide module bypass line **537** fluidly connecting valve **530** in the zirconium oxide inlet line to valve **531** in the zirconium oxide effluent line **538.** Valves **530** and **531** can direct fluid through the zirconium oxide module bypass line **537** and into zirconium oxide effluent line **538.** Zirconium oxide module bypass line **537** can convey fluid directly from the base source **508** to the zirconium oxide effluent line **538** to neutralize the zirconium phosphate effluent without the need to simultaneously recharge a zirconium oxide module **504.** Alternatively, zirconium oxide sorbent module inlet **535** can be fluidly connected to zirconium oxide sorbent module outlet **536.** Multiple sensors can be included in the zirconium oxide recharging flow path **502** to monitor fluid concentration. For example, conductivity sensor **532** can monitor concentrations of the zirconium oxide recharging fluid; pressure sensor **534** can monitor pressure in the zirconium oxide inlet line and to detect leaks or occlusions. Flow sensor **533** can determine the flow rate of the fluid through the zirconium oxide inlet line and be used to control zirconium oxide pumps **526** and **527.** A static mixer (not shown) can be positioned upstream of the zirconium oxide module **504** and mix solutions prior to entering the zirconium oxide module **504.** A heater and heat exchanger (not shown) can be positioned in the zirconium oxide recharging flow path **502** to heat fluids prior to entering zirconium oxide module **504.** Heating fluid in the zirconium oxide recharging flow path **502** can reduce recharging times and allow disinfection with a base solution, such as sodium hydroxide. Heating the fluid also allows for reduced disinfection time with a disinfectant source. A zirconium oxide rinse loop (not shown) can also be included to bypass the heater and heat exchanger during flushing.

Effluent from zirconium phosphate recharging flow path **501** can neutralize, either completely or in part, the effluent from zirconium oxide recharging flow path **502,** and vice versa. Zirconium phosphate effluent line **539** can be fluidly connected to zirconium oxide effluent line **538** at an effluent line junction **540** joining drain line **545,** which fluidly connects to drain **547.** Static mixer **546** can be used at or downstream of the effluent line junction **540** to mix zirconium phosphate effluent with zirconium oxide effluent.

Zirconium phosphate effluent line **539** and zirconium oxide effluent line **538** can be connected to a common reservoir for storage and disposal of the combined effluent. The common reservoir receives and collects the zirconium phosphate and zirconium oxide effluents together. The collected effluents can be drained after appropriate volumes of each effluent have been added to achieve neutralization. A common reservoir can allow for neutralization of the zirconium phosphate and zirconium oxide effluents without synchronizing the recharging processes. A single common reservoir can be sized to support multiple recharge stations.

Alternatively, the two fluid streams may be mixed through fluid line mixing at the effluent line junction **540.** Flow sensor **541** and conductivity sensor **542** can be placed in zirconium phosphate effluent line **539** to measure the flow rate and composition of the zirconium phosphate effluent. Flow sensor **544** and conductivity sensor **543** can be positioned in the zirconium oxide effluent line **538** to measure the flow rate and composition of the zirconium oxide effluent. Data from flow sensors **541** and **544** and conductivity sensors **542** and **543** can determine if the combined effluent in drain line **545** is safe for disposal into a drain. One non-limiting example of safe is an effluent having a pH in the range of 5-9. Either zirconium phosphate effluent line **539** or zirconium oxide effluent line **538** can be connected simultaneously or independently to a waste reservoir (not shown) for disposal. Additional pH or conductivity sensors can be positioned downstream of the static mixer **546** to monitor and ensure safe disposal. Drain line **545** can also be connected to a common waste reservoir for storage and disposal of effluent. The common reservoir receives and collects the zirconium phosphate and zirconium oxide effluents together. The collected effluents can be drained after appropriate volumes of each effluent have been added to achieve neutralization. A common waste reservoir advantageously allows for neutralization of the zirconium phosphate and zirconium oxide effluents without synchronizing the recharging processes. Static mixer **546** is unnecessary when a common reservoir is used.

Brine source **506,** disinfectant source **507,** and base source **508** can have filter **548,** filter **549,** and filter **550,** respectively to remove particulate matter. The one or more filters can remove particulate matter before fluid enters the zirconium oxide recharging flow path **502** or zirconium phosphate recharging flow path **501.** Water source **505** can have microbial filter **556** to remove microbes from the water before entering the flow paths. In FIG. 5C, the dashed line **553** represents a recharger housing. The fluid sources can be external to the recharger housing and fluidly connected to the lines located inside of the recharger housing. Alternatively, the fluid sources described can instead be housed within the recharger.

During recharging, fluid can be passed through the zirconium phosphate module **503** and/or the zirconium oxide module **504** opposite to a flow direction used during dialysis. For example, zirconium phosphate sorbent module inlet **524** can be used as the zirconium phosphate sorbent module outlet during dialysis, and zirconium phosphate sorbent module outlet **525** can be used as the zirconium phosphate sorbent module inlet during dialysis in FIG. 5B. Similarly, zirconium oxide sorbent module inlet **535** can be used as the zirconium phosphate sorbent module outlet during dialysis, and zirconium oxide sorbent module outlet **536** can be used as the zirconium phosphate sorbent module inlet during dialysis. Pumping the recharging fluid through the modules in the opposite direction relative to dialysis can improve the efficiency of the recharging process.

The zirconium phosphate recharging flow path **501** or zirconium oxide recharging flow path **502** can independently recharge zirconium phosphate or zirconium oxide. For example, a single flow path fluidly connecting zirconium phosphate module **503** of FIG. 5B via valve **512** and valve **513** to each of the water source **505,** brine source **506,** and disinfectant source **507** can independently recharge the zirconium phosphate module **503.** Similarly, a single flow path fluidly connecting zirconium oxide module **504** of FIG. 5C via valve **528** and valve **529** to each of the water source **505,** disinfectant source **507,** and base source **508** can independently recharge the zirconium oxide module **504.**

The water source **505,** brine source **506,** disinfectant source **507,** and base source **508** can recharge one or more reusable sorbent module of various sizes. The amount of water, brine, disinfectant, and base depends on the concentration of each of the recharging solutions, the size of the reusable sorbent modules, the amount of cations/anions removed, and the flow rate used to pass the solutions through the reusable modules. The amount of brine solution required can depend on the temperature to which the brine solution is heated. For example, a brine solution having between 2.5 M and 4.9 M sodium chloride, between 0.3 M and 1.1 M sodium acetate, and between 0.2 M and 0.8 M acetic acid at between 70°C and 90°C requires between 4.2-6.2 L of brine to recharge a zirconium phosphate module containing between 2 kg and 3.2 kg of zirconium phosphate loaded with 2 to 3 moles of ammonium, calcium, magnesium and potassium. The brine solution should have a volume of at least between 4.2 and 6.2 L and delivered at a flow rate of between 100 and 300 mL/min. A single brine source can be connected to multiple rechargers, or can recharge multiple zirconium phosphate modules in a single recharger. The brine source can have a significantly larger volume from 1-100X or greater to ensure that the brine source toned not be refilled each time a zirconium phosphate is recharged. For a zirconium oxide module having between 220 and 340 g of zirconium oxide loaded with 200 mmols of phosphate, a base source having between 0.5 and 2.0 M sodium hydroxide and a flow rate between 30 and 150 mL/min requires between 1 and 4 L of base. The base source can be at least between 1 and 4 L in volume. For recharging multiple zirconium oxide modules, a larger base source can be used.

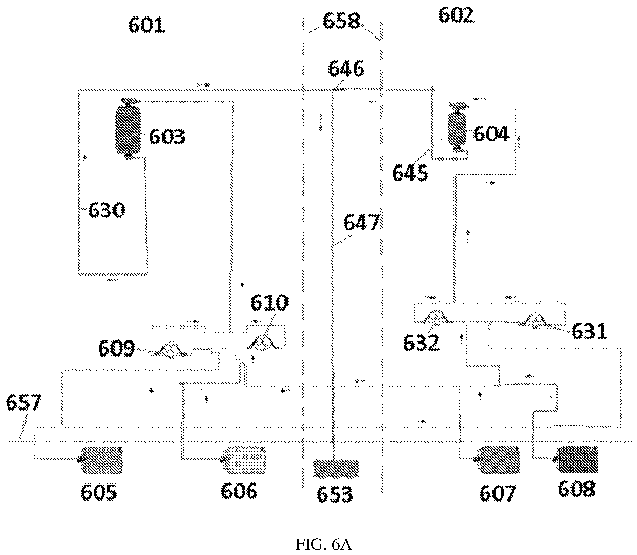

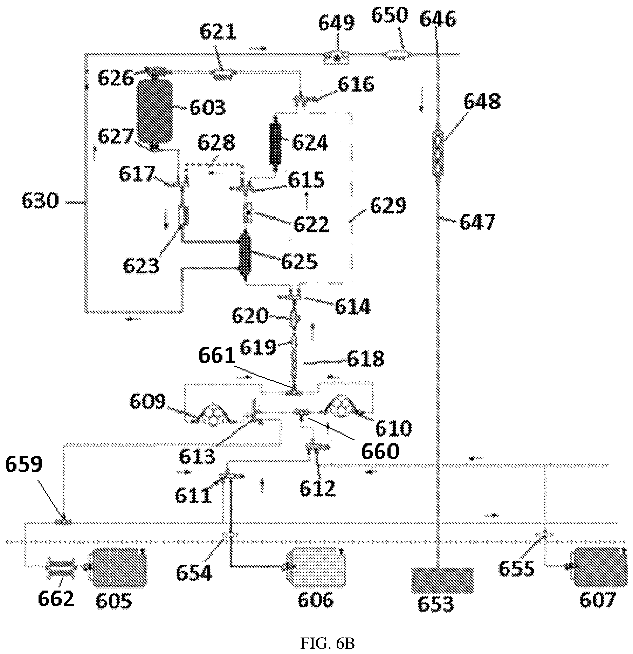

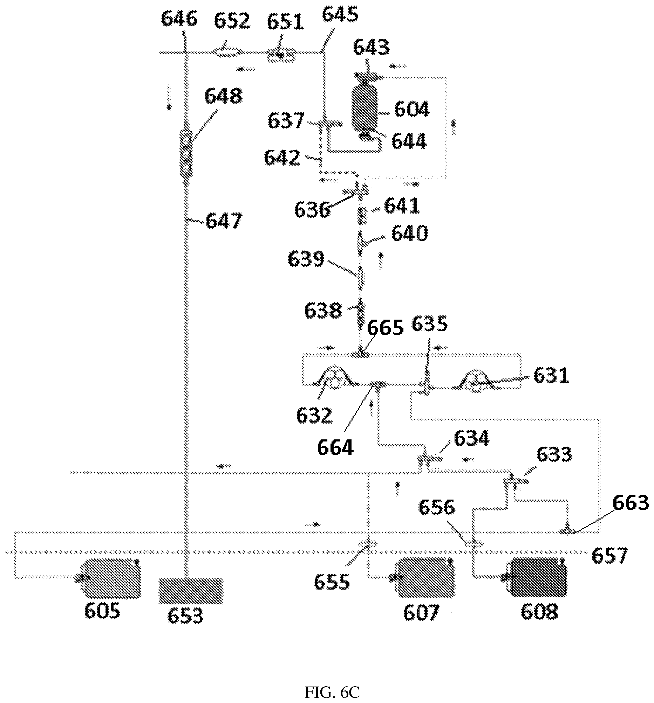

FIG. 6A is a generalized view of a recharging flow path having a zirconium phosphate recharging flow path **601** containing a zirconium phosphate module **603** and a zirconium oxide recharging flow path **602** containing a zirconium oxide module **604.** FIG. 6B illustrates a detailed view of zirconium phosphate recharging flow path **601** on the zirconium phosphate side of line **658,** and FIG. 6C illustrates a detailed view of zirconium oxide recharging flow path **602** on the zirconium oxide side of line **658.** The valves, pumps and static mixers illustrated in FIG.'s 6B and 6C can allow for inline mixing of the recharging fluids. In FIG. 6A, the zirconium phosphate recharging flow path **601** and/or zirconium oxide recharging flow path **602** can be simultaneously or independently connected to a water source **605,** a brine source **606,** a disinfectant source **607,** and a base source **608.** Because recharging of the zirconium phosphate in a zirconium phosphate module **603** can require water, brine, and disinfectant, and because recharging of zirconium oxide in zirconium oxide module **604** can also require water, base, and disinfectant, the water source, **605,** the brine source **606,** and the disinfectant source **607** can be jointly connected to the zirconium phosphate recharging flow path **601,** and the water source **605,** the disinfectant source **607,** and the base source **608** can be jointly connected to the zirconium oxide recharging flow path **602.**

In FIG. 6A, zirconium phosphate recharging flow path **601** and zirconium oxide recharging flow path **602** can mix chemicals in-line to create the recharging solutions. Any one of disinfectant source **607,** brine source **606,** and base source **608** can contain solutions having concentrations over the concentration of the components to be used in recharging the reusable modules. Water source **605** can dilute the disinfectant, brine, and base from the fluid sources prior to recharging. In FIG. 6B, zirconium phosphate pump **610** can pump disinfectant into the zirconium phosphate module **603** with in-line mixing of concentrated disinfectant from disinfectant source **607** from valve **612** through junctions **660** and **661** and into static mixer **618.** Concurrently, zirconium phosphate pump **609** can pump water through junction **659** and valve **613** and into static mixer **618** from water source **605.** Alternatively, the concentrated disinfectant and water can be mixed through fluid line mixing at the junction of the two fluid lines. The zirconium phosphate pumps **609** and **610** can pump a disinfectant solution having a specified concentration and composition to disinfect the zirconium phosphate module **603** via valves **612** and **613.** The disinfectant solution can flow from static mixer **618** through valve **614** to valve **616** and then a resulting solution can flow into the zirconium phosphate module **603** through zirconium phosphate sorbent module inlet **626.** Fluid can exit zirconium phosphate module **603** through zirconium phosphate sorbent module outlet **627** into zirconium phosphate effluent line **630.** After disinfection of zirconium oxide module **603,** zirconium phosphate pumps **609** and **610** can pump water from water source **605** into zirconium phosphate module **603.** For example, zirconium phosphate pump **609** can pump water through valve **613** to zirconium phosphate module **603** while zirconium phosphate pump **610** can pump water through valves **611** and **612** to zirconium phosphate module **603.** Alternatively, zirconium phosphate pump **609** can pump water through valves **611, 612,** and **613** while zirconium phosphate pump **610** pumps water through valves **611** and **612.** During recharging, zirconium phosphate pumps **609** and **610** can pump brine through valve **611** to valve **612** from brine source **606** into static mixer **618.** If a concentrated brine solution is being used, zirconium phosphate pumps **609** and/or **610** can pump water from water source **605** to static mixer **618** to dilute the brine solution and generate a brine solution having a proper solute concentration for recharging the zirconium phosphate. After pumping brine through the zirconium phosphate module **603,** zirconium phosphate pump **609** can pump water through valves **611, 612** and **613** while zirconium phosphate pump **610** can pump water through valve **611** and **612.**

The zirconium phosphate recharging flow path **601** of FIG. 6B can have a heater **624** and heat exchanger **625.** One or more heat exchangers and one or more heaters can be used. The brine solution can be heated by the heater **624** upstream of the zirconium phosphate module **603.** Heat exchanger **625** can utilize the heat from brine exiting the zirconium phosphate module **603** to heat the incoming brine solution upstream of heater **624** to reduce the burden on heater **624.** As described, the zirconium phosphate recharging flow path **601** can also have an optional zirconium phosphate module bypass line **628** fluidly connecting valve **615** in the zirconium phosphate inlet line to valve **617** in the zirconium phosphate effluent line **630.** The zirconium phosphate module bypass line **628** can neutralize the zirconium oxide effluent with brine even if the zirconium phosphate module **103** is not being recharged. Zirconium phosphate recharging flow path **601** can have a rinse loop **629** connecting valve **614** upstream of the heater **624** and heat exchanger **625** to valve **616** to bypass heater **624** and heat exchanger **625** to rinse brine out of the zirconium phosphate module **603.**

Various sensors can be included in the zirconium phosphate recharging flow path **601** to ensure fluid parameters are within acceptable ranges. In FIG. 6B, conductivity sensor **619** can be placed downstream of static mixer **618** to ensure mixing and specified recharging fluid concentrations. Pressure sensor **620** can measure the fluid pressure and to identify leaks or occlusions. Flow sensor **622** can determine the flow rate of the fluid entering the zirconium phosphate module **603** and control zirconium phosphate pumps **609** and **610.** Temperature sensor **621** can determine if the recharging fluid is a proper temperature range upon entering zirconium phosphate module **603** and relay data to a processor (not shown) that can control heater **624.** Temperature sensor **623** can determine the temperature of the zirconium phosphate effluent prior to entering heat exchanger **625.** Other sensor arrangements, including any number of conductivity, pressure, flow, and temperature sensors can be used.

In FIG. 6C, zirconium oxide pump **632** can pump disinfectant from disinfectant source **607** through valve **634** and into static mixer **638** to disinfect the zirconium oxide module **604** in zirconium oxide recharging flow path **602.** Zirconium oxide pump **631** can pump water from water source **605** through valve **635** to static mixer **638** to dilute the disinfectant from disinfectant source **607** to provide in-line mixing of the disinfectant solution. The diluted disinfectant can then be pumped through valve **636** to zirconium oxide sorbent module inlet **643** and into zirconium oxide module **604.** Effluent from the zirconium oxide module **604** can exit through zirconium oxide sorbent module outlet **644** and into zirconium oxide effluent line **645.** After disinfection, the disinfectant can be rinsed from the zirconium oxide module **604** by pumping water from water source **605** through valve **635** to zirconium oxide module **604** by zirconium oxide pump **631** while zirconium oxide pump **632** pumps water through valves **633** and **634** to zirconium oxide module **604.** Alternatively, zirconium oxide pump **631** can pump water through valves **633, 634,** and **631,** while zirconium oxide pump **632** pumps water through valves **633** and **634.** To recharge zirconium oxide module **604,** zirconium oxide pump **632** can pump base from base source **608** through valves **633** and **634** through junctions **664** and **665** to static mixer **638.** Water from water source **605** can be pumped by zirconium oxide pump **631** through junctions **663** and **665** into static mixer **638** to dilute the base by in-line mixing. Alternatively, the water and base can be mixed through fluid line mixing at the junction of the two fluid lines. Alternatively, the base can be pre-set using specified amounts of base in pre-packaged packets or containers. Diluted base can flow through the zirconium oxide recharging flow path **602** and through zirconium oxide module **604.** The zirconium oxide module **604** can be rinsed any numbers of times, as needed, by introducing water from water source **605** to the zirconium oxide module **604.** The zirconium oxide recharging flow path **602** can also have a zirconium oxide module bypass line **642** that fluidly connects valve **636** to valve **637** in the zirconium oxide effluent line **645** to bypass zirconium oxide module **604.** In this way, zirconium phosphate effluent can be neutralized with a base solution even if the zirconium oxide module **604** is not being recharged. A heater and heat exchanger (not shown) can be positioned in the zirconium oxide recharging flow path **602** to heat fluids prior to entering zirconium oxide module **604.** A zirconium oxide rinse loop (not shown) can also be included to bypass the heater and heat exchanger. Similarly, the zirconium oxide recharging flow path **602** can also have sensors for measurement and control over the recharging process. In FIG. 6C, a conductivity sensor **639** can be placed downstream of static mixer **638** to ensure that diluted recharging solutions have a desired concentration. Pressure sensor **640** can detect the pressure in the zirconium oxide recharging flow path **602** to detect leaks or occlusions. Flow sensor **641** can detect the flow rate of fluid in the zirconium oxide recharging flow path **602** and can control zirconium oxide pumps **631** and **632.**

As shown in FIG. 6A, the present invention can provide in-line neutralization of the effluent from each of the zirconium phosphate recharging flow path **601** and zirconium oxide recharging flow path **602.** The zirconium phosphate effluent line **630** can be fluidly connected to zirconium oxide effluent line **645** at effluent line junction **646** and fluidly connected to drain line **647.** As shown in FIG.'s 6B and 6C, a static mixer **648** can be positioned at or downstream of the effluent line junction **646** to ensure mixing of the effluents from the zirconium phosphate recharging flow path **601** and zirconium oxide recharging flow path **602.** The combined effluent can be passed through the drain line **647** to drain **653,** a common waste reservoir (not shown), or separate waste reservoirs. A conductivity sensor **650** as shown in FIG. 6B in zirconium phosphate effluent line **630** and a conductivity sensor 652 as shown in FIG. 6C in zirconium oxide effluent line **645** can determine the composition of the effluents. Flow sensor **649** in zirconium phosphate effluent line **630** of FIG. 6B and flow sensor **651** in zirconium oxide effluent line **645** of FIG. 6C can be used simultaneously or independently to measure the flow rates of each of the effluents. Determining the composition of the effluent fluids as well as the respective flow rates using one or more sensors described can monitor the system function and ensure that the combined effluent in drain line **647** is safe for disposal or storage.

Brine source **606,** disinfectant source **607,** and base source **608** can have filter **654,** filter **655,** and filter **656,** respectively to remove particulate matter prior to entering zirconium phosphate recharging flow path **601** or zirconium oxide recharging flow path **602.** The filters can also act as inline mixers to mix the solutions. Water source **605** can have microbial filter **662** to remove microbes from the water. Brine source **606,** disinfectant source **607,** and base source **608** can be housed outside of a recharger housing denoted by line **657.** The brine solution, disinfectant solution, and base solution can be generated through in-line mixing as described. Alternatively, pre-mixed solutions, concentrates, or infusates can be introduced into brine source **606,** disinfectant source **607,** and base source **608** and delivered to zirconium phosphate recharging flow path **601** or zirconium oxide recharging flow path **602.** For example, the brine solution in brine source **606** can be pre-mixed or provide in pre-packaged amounts in the proper concentrations and introduced into brine source **606,** disinfectant source **607,** and base source **608.**

In-line mixing can provide higher concentrations of solutes, lower fluid volumes required by the system, and physically smaller fluid reservoirs. The fluids should have suitable concentrations for use in the zirconium phosphate recharging flow path **601** or zirconium oxide recharging flow path **602.** For example, an initially high source of peracetic acid can be used in a concentration of between 20% and 40%. The zirconium phosphate recharging flow path **601** of FIG. 6B can dilute the peracetic acid or other disinfectant in the disinfectant source by a factor of 20:1 to 40:1 to generate an acidic recharging solution having a concentration between 0.5 % and 2%. The initial disinfectant concentration can be any concentration greater than 1%. Similarly, the base solution can be sodium hydroxide having an initial concentration between 14M and 22M. The zirconium oxide recharging flow path **602** of FIG. 6C can dilute the base solution by 18:1 to 22:1 to generate a base solution having a concentration between 0.8 and 1.0 M. The initial base solution concentration can be any concentration greater than or equal to 0.5 M. The brine solution can also be diluted in-line to generate a brine solution having a proper recharging concentration. The brine source **606** of FIG. 6A can be one or more reservoirs. For example, an acetic acid source, a sodium acetate source and a sodium chloride source can each be connected in place of single brine source **606.** Alternatively, an acetic acid source, a base source, and a sodium chloride source can be connected in place of the single brine source **606** with mixing of the base and acetic acid to generate the sodium acetate. The individual components can be added to the zirconium phosphate recharging flow path **601** in the proper ratios to generate the recharging brine.

The chemicals used in the recharging process can be packaged and shipped in any form. The chemicals can be packaged and shipped as solutions, either in proper concentrations for use in recharging or with higher concentrations for use in in-line mixing. In any embodiment, the chemicals may be packaged and shipped in pure form, such as 100% acetic acid or solid sodium chloride, sodium acetate, or sodium hydroxide.

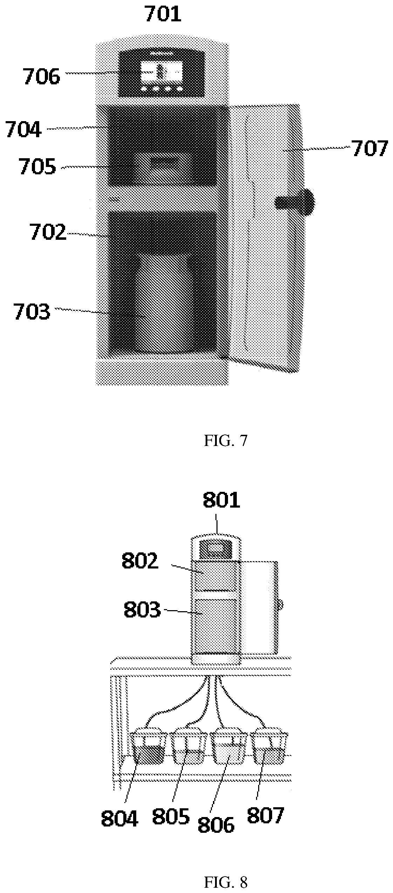

A recharger can be configured as shown in FIG. 7. The recharger **701** includes a fist receiving compartment **702** for receiving a reusable zirconium phosphate module **703** and a second receiving compartment **704** for receiving a zirconium oxide module **705.** Fluid connections (not shown in FIG. 7) connect to the top and bottom of the sorbent modules **703** and **705** for passing recharging fluids into, through, and out of the reusable sorbent modules **703** and **705.** As described, the recharging fluids replace ions bound to the sorbent materials during dialysis with new ions, recharging the sorbent material within the sorbent modules for reuse in sorbent dialysis. The recharger **701** can be configured to concurrently recharge a zirconium phosphate module **703** and a zirconium oxide module **705,** or to independently recharge either a zirconium phosphate module **703** or a zirconium oxide module **705** with neutralization of the effluents of each of the sorbent modules. A user interface **706** is provided to start or control the recharging process by the user. The user interface **706** also provides the status of the recharging process to the user, such as the times of completion of each recharging step, or a time until the recharging process is complete. User interface **706** also provides alert messages if any problems are detected during recharging, such as leaks, occlusions, pump failures, or mismatched chemicals. A door **707** on the recharger **701** controls access to the receiving compartments **702** and **704** during operation. Rechargers with any number of receiving compartments for recharging any number of zirconium oxide and/or zirconium phosphate sorbent modules can be constructed. For example, a recharger with two zirconium phosphate receiving compartments and two zirconium oxide receiving compartments can be similarly constructed. The rechargers can have 1, 2, 3, 4, 5, 6, or more receiving compartments, each capable of receiving zirconium oxide or zirconium phosphate sorbent modules.

FIG. 8 illustrates non-limiting embodiment of a recharger set up for recharging zirconium oxide and zirconium phosphate, either concurrently or independently. To recharge the sorbent materials, one or more recharging fluids are passed through the reusable sorbent modules. As shown in FIG. 8, the recharger **801** is fluidly connected to one or more recharging fluid sources, such as water source **804,** brine source **805,** disinfectant source **806,** and base source **807.** The recharger has a zirconium phosphate receiving compartment **803** and a zirconium oxide receiving compartment **802.** The recharger also has one or more pumps and valves (not shown in FIG. 8) for selectively delivering the recharging fluids from the fluid sources to the reusable modules. As shown in FIG. 8, the recharging fluid sources are housed external to the recharger **801.** Alternatively the recharging fluid sources can be housed within the recharger **801.** A drain line (not shown) is also connected to the recharger **801** for disposal of waste fluids exiting the reusable modules. The drain line is fluidly connected to a drain, or alternatively, the drain line can be fluidly connected to one or more waste reservoirs for storage and later disposal. The effluents from the zirconium oxide module and zirconium phosphate module are each delivered to the drain line for neutralization of the recharging fluids.

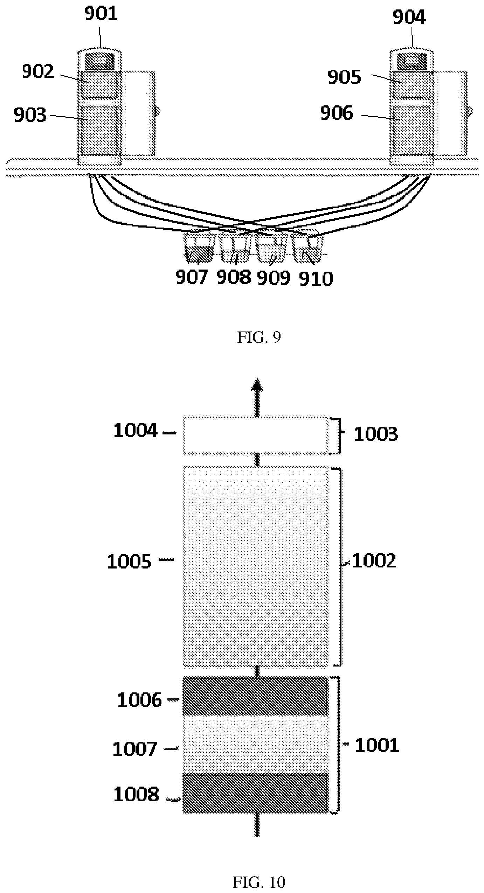

As illustrated in FIG. 9, multiple rechargers can be chained together and connected to a single set of fluid sources for sharing of infrastructure. A first recharger **901** having a zirconium phosphate receiving compartment **903** and zirconium oxide receiving compartment **902** is fluidly connected to water source **907,** brine source **908,** disinfectant source **909,** and base source **910.** A second recharger **904** having a zirconium oxide receiving compartment **905** and zirconium phosphate receiving compartment **906** is also fluidly connected to the same water source **907,** brine source **908,** disinfectant source **909,** and base source **910.** Any number of rechargers can be connected to a common set of fluid sources, including 2, 3, 4, 5, 6 or more rechargers, each fluidly connected to a single set of fluid sources and a single set of waste reservoirs. Connecting multiple rechargers to a single set of fluid sources saves space and materials and simplifies recharging multiple sets of reusable modules in a clinic or hospital setting. Each of the rechargers may include a separate drain line and/or separate waste reservoirs, or each recharger may be fluidly connected to a common drain line. The drain line can also be fluidly connected to any one of a drain, a common reservoir, or combinations thereof. Each of the connected rechargers can have separate heaters for heating the brine and/or disinfectant solutions, or centralized heaters can be included, with centralized heating of the shared solutions.

The rechargers can be used in any setting, including a clinic, at home, or in a mobile setting. In any setting, the rechargers can use a water tank or any other source of potable or deionized water. For use in a mobile setting, vans or trucks can carry the rechargers, the disinfectant source, the brine solution, the base solution, and optionally the water, to a location for recharging. For at home use, the brine solution, disinfectant solution, base solution, and optionally the water, may be prepackaged and shipped to a patient. The patient can connect each of the sources to the recharger to allow recharging and reuse of the sorbent modules in dialysis. As described, the rechargers can provide for inline mixing of chemicals, reducing the amount of chemicals required to be moved for use in a mobile setting. Inline mixing of chemicals allows for a smaller amount of concentrated solutions to be moved to a location in a mobile or at home setting, and water from a local water source, such as municipal drinking water, can dilute the disinfectant, base, and/or brine inline. Alternatively, a deionized or purified water source can be provided in a mobile setting. Effluent from the sorbent modules can be collected and neutralized inline for immediate disposal in any drain, or can be collected for later neutralization and disposal offline. The ability to neutralize and dispose of the combined effluents in a drain allow for easier use in an at home or mobile setting, without the need for large waste reservoirs and further treatment.

A non-limiting embodiment of a reusable sorbent cartridge having modules that can be separated and recharged by systems and methods of the present invention is shown in FIG. 10. The sorbent cartridge can be separated into reusable modules to facilitate recharging of one or more sorbent materials. In FIG. 10, the sorbent cartridge has a first sorbent module **1001,** a second sorbent module **1002,** and a third sorbent module **1003.** The first module **1001** can have a layer of activated carbon **1008,** a layer of alumina and urease **1007,** and a second layer of activated carbon **1006.** The activated carbon can remove many non-ionic solutes from the dialysate. The urease catalyzes the conversion of urea in the dialysate into ammonium ions. The alumina can serve as a support for the urease. The second layer of activated carbon **1006** can capture any urease that migrates out of alumina and urease layer **1007** prior to exiting the first module **1001.** The first module **1001** can be a single use module, or can be a multiple use module with replenishment of the urease. The second module **1002** can have zirconium phosphate **1005.** After dialysis, zirconium phosphate **1005** will contain bound potassium, calcium, magnesium, and ammonium ions, which can be replaced with sodium and hydrogen ions by the recharging process described herein. Third module **1003** can contain zirconium oxide **1004.** After use, the zirconium oxide **1004** will contain bound phosphate, fluoride and other anions, which can be replaced with hydroxide anions through the recharging process described herein. The flow direction of flow of dialysate through the sorbent cartridge is shown by the arrow in FIG. 10. The recharging solutions can also flow through the reusable sorbent modules in an opposite direction to improve the efficiency of the recharging process.

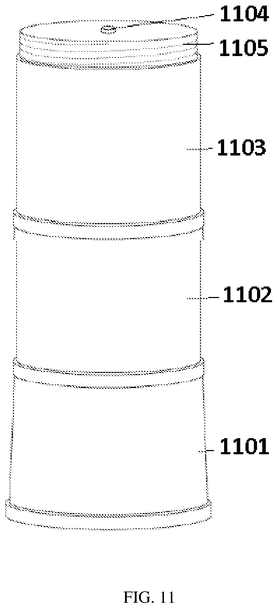

FIG. 11 illustrates another non-limiting example of a modular sorbent cartridge that can be used in the recharging process described herein. The modular sorbent cartridge can be separated into discrete modules including a first module **1101,** a second module **1102,** and a third module **1103** connected together to form a sorbent cartridge. The first module **1101** can contain activated carbon, urease, and alumina; the second module **1102** can contain zirconium phosphate; and the third module **1103** can contain zirconium oxide. One of skill in the art will understand that the modular sorbent cartridge illustrated in FIG. 11 is for illustrative purposes only, and modifications to the sorbent cartridge can be made within the scope of the invention. Alternatively, the sorbent modules can be independent with fluid lines connecting each of the sorbent modules for dialysis. During dialysis, dialysate can enter the sorbent cartridge through the bottom of first module **1101,** travel through modules **1101, 1102,** and **1103,** and exit through fluid outlet **1104.** The fluid outlet **1104** can connect to the rest of the dialysate flow path. Threaded portion **1105** on module **1103** can be used in connecting modules to each other, to the dialysate flow path, or to the recharger as described herein. The threaded portion **1105** can be included on any of the sorbent modules. Other connection types suitable for secured fluid connection in dialysis known in the art is contemplated by the invention. For example, fluid lines can be clamped directly onto fluid outlet **1104.** After dialysis, a user can disconnect the sorbent modules for disposal of single use modules and for recharging of the reusable modules.

One skilled in the art will understand that various combinations and/or modifications and variations can be made in the described systems and methods depending upon the specific needs for operation.

## Claims

1. A sorbent recharger, comprising:
at least a first receiving compartment for a first sorbent module (604); the first receiving compartment having a first sorbent module inlet (643) and a first sorbent module outlet (644);
a first inlet line fluidly connected to the first sorbent module inlet (643);
a first effluent line (645) fluidly connected to the first sorbent module outlet;
a disinfectant source (607), a base source (608), a water source (605), and a brine source (606); wherein any one of one of the disinfectant source (607), base source (608), water source (605), brine source (606), and combinations thereof is fluidly connected to the first inlet line; and wherein at least one of the disinfectant source (607), base source (608), water source (605) and brine source (606) is fluidly connected to the first effluent line at a junction(665); and a static mixer (638) at or downstream of the junction (665);
wherein:
a) the water source (605), disinfectant source (607), and brine source (606) are fluidly connected to the first inlet line; and wherein at least the water source (605) and base source (608) are fluidly connected to the first effluent line; or
b) the water source (605), disinfectant source (607), and base source (608) are fluidly connected to the first inlet line; and wherein at least the water source (605) and brine source (606) are fluidly connected to the first effluent line (645).

2. The sorbent recharger of claim 1, wherein the sorbent recharger comprises a second receiving compartment for a second reusable sorbent module (603); the second receiving compartment comprising a second sorbent module inlet and a second sorbent module outlet;
a second inlet line fluidly connected to the second sorbent module inlet;
a second effluent line (630) fluidly connected to the second sorbent module outlet;
wherein at least one of the disinfectant source (607), base source (608), water source (605), and brine source (606) is fluidly connected to the second inlet line; and wherein the second effluent line (630) is fluidly connected to the junction (665).

3. The sorbent recharger of claim 1 or claim 2, further comprising a conductivity sensor (619, 639) positioned in the first effluent line; preferably further comprising a conductivity sensor (619, 639) positioned in each of the first effluent line (645) and second effluent line (630).

4. The sorbent recharger of any preceding claim, further comprising at least a first pump (609, 610) in the first inlet line for pumping fluid from the disinfectant source (607), base source (608), water source (605), and/or brine source (606) to the first sorbent module inlet (643); and
at least a second pump (631, 632) for pumping fluid from the disinfectant source (607), base source (608), water source (605), and/or brine source (606) to the junction (665);
preferably wherein the at least second pump (631, 632) is in the second inlet line for pumping fluid from the disinfectant source (607), base source (608), water source (605), and/or brine source (606) to the second sorbent module inlet; and
the sorbent charger further comprises a controller for controlling the first pump and second pump (631, 632).

5. The sorbent recharger of claim 4, wherein the controller is configured to control the first pump (609, 610) to pump fluid from the brine source (606) to the first sorbent module inlet (643); and to control the second pump (631, 632) to pump fluid from the base source (608) to the second sorbent module inlet concurrently.

6. The sorbent recharger of claim 2, further comprising a least one module bypass line (628, 642), wherein the module bypass line fluidly connects either: the first inlet line to the first effluent line; or the second inlet line to the second effluent line;
and/or wherein the first sorbent module inlet is fluidly connectable to the first sorbent module outlet and/or the second sorbent module inlet is fluidly connectable to the second sorbent module outlet.

7. The sorbent recharger of any of claims 1 to 8, further comprising a pH sensor downstream of the static mixer.

8. A method, comprising the steps of:
pumping a brine solution through a first sorbent module (603) containing zirconium phosphate in a sorbent recharger according to claim 1;
pumping the resulting solution to a static mixer (638) or common reservoir; and concurrently pumping a base solution to the static mixer or common reservoir.

9. The method of claim 8, wherein the step of pumping the base solution to the static mixer (638) or common reservoir comprises the step of pumping the base solution through a second sorbent module (604), the second sorbent module containing zirconium oxide;
and/or wherein the step of pumping the base solution to the static mixer comprises the step of pumping the base solution through a module bypass line (628, 642);
and/or further comprising the step of pumping fluid from the static mixer (638) to a drain (653); and/or further comprising the step of sensing a conductivity or pH in an effluent of the sorbent module (603) containing zirconium phosphate with a conductivity sensor (619, 639), a pH sensor, or combinations thereof; and wherein the step of concurrently pumping a base solution to the static mixer (638) is carried out when the conductivity sensor (619, 639) senses an increase in conductivity, the pH sensor sensing a decrease in pH, or combinations thereof, in the effluent of the sorbent module (603) containing zirconium phosphate.

10. A method comprising the steps of:
pumping a base solution through a first sorbent module (604) containing zirconium oxide in a sorbent recharger according to claim 1;
pumping a resulting solution to a static mixer (638) or common reservoir; and concurrently pumping a brine solution to the static mixer (638) or common reservoir.

11. The method of claim 10, wherein the step of pumping the brine solution to the static mixer (638) or common reservoir comprises the step of pumping the brine solution through a second sorbent module (603), the second sorbent module containing zirconium phosphate; and/or wherein the step of pumping the brine solution to the static mixer (638) or common reservoir comprises the step of pumping the brine solution through a module bypass line (628, 642).

12. The method of claim 10, further comprising the step of sensing a conductivity or pH in an effluent of the sorbent module (604) containing zirconium oxide with a conductivity sensor (619, 639), a pH sensor, or combinations thereof; and wherein the step of concurrently pumping a brine solution to the static mixer (638) is carried out when the conductivity sensor (619, 639) senses an increase in conductivity, the pH sensor sensing an increase in pH, or combinations thereof, in the effluent of the sorbent module (604) containing zirconium oxide.

13. The method of any of claims 8 to 12, further comprising: determining a pH in the fluid downstream of the static mixer (638) with a sensor; and pumping the fluid from the static mixer (638) to a drain (653) if the pH of the fluid is between 5 and 9.

## Patentansprüche

1. Sorptionsmittelladegerät, umfassend:
mindestens ein erstes Aufnahmefach für ein erstes Sorptionsmittelmodul (604);
wobei das erste Aufnahmefach einen ersten Sorptionsmittelmoduleinlass (643) und einen ersten Sorptionsmittelmodulauslass (644) aufweist;
eine mit dem ersten Sorptionsmittelmoduleinlass (643) in Fluidverbindung stehende, erste Einlassleitung;
eine mit dem ersten Sorptionsmittelmodulauslass in Fluidverbindung stehende, erste Abflussleitung (645);
eine Desinfektionsmittelquelle (607), eine Basenquelle (608), eine Wasserquelle (605) und eine Salzlösungsquelle (606);
wobei die Desinfektionsmittelquelle (607), Basenquelle (608), Wasserquelle (605), Salzlösungsquelle (606) oder Kombinationen davon mit der ersten Einlassleitung in Fluidverbindung stehen; und
wobei die Desinfektionsmittelquelle (607), Basenquelle (608), Wasserquelle (605) und/oder Salzlösungsquelle (606) an einer Verzweigungsstelle (665) mit der ersten Abflussleitung in Fluidverbindung stehen; und
einen statischen Mischer (638) an der Verzweigungsstelle (665) oder der ihr nachgelagert ist;
wobei:
a) die Wasserquelle (605), Desinfektionsmittelquelle (607) und die Salzlösungsquelle(606) mit der ersten Einlassleitung in Fluidverbindung stehen; und wobei mindestens die Wasserquelle (605) und die Basenquelle (608) mit der ersten Abflussleitung in Fluidverbindung stehen; oder
b) die Wasserquelle (605), Desinfektionsmittelquelle (607) und Basenquelle (608) mit der ersten Einlassleitung in Fluidverbindung stehen; und wobei mindestens die Wasserquelle (605) und die Salzlösungsquelle (606) mit der ersten Abflussleitung (645) in Fluidverbindung stehen.

2. Sorptionsmittelladegerät nach Anspruch 1, wobei das Sorptionsmittelladegerät ein zweites Aufnahmefach für ein zweites wiederverwendbares Sorptionsmittelmodul (603) umfasst;
das zweite Aufnahmefach einen zweiten Sorptionsmittelmoduleinlass und einen zweiten Sorptionsmittelmodulauslass umfasst;
eine zweite Einlassleitung mit dem zweiten Sorptionsmittelmoduleinlass in Fluidverbindung steht;
eine zweite Abflussleitung (630) mit dem zweiten Sorptionsmittelmodulauslass in Fluidverbindung steht;
wobei die Desinfektionsmittelquelle (607), Basenquelle (608), Wasserquelle (605) und/oder Salzlösungsquelle (606) mit der zweiten Einlassleitung in Fluidverbindung steht; und
wobei die zweite Abflussleitung (630) mit der Verzweigungsstelle (665) in Fluidverbindung steht.

3. Sorptionsmittelladegerät nach Anspruch 1 oder Anspruch 2, ferner umfassend einen Leitfähigkeitssensor (619, 639), der in der ersten Abflussleitung angeordnet ist;
vorzugsweise ferner umfassend einen Leitfähigkeitssensor (619, 639), der sowohl in der ersten Abflussleitung (645) als auch der zweiten Abflussleitung (630) angeordnet ist.

4. Sorptionsmittelladegerät nach einem der vorhergehenden Ansprüche, ferner umfassend mindestens eine erste Pumpe (609, 610) in der ersten Einlassleitung zum Pumpen von Fluid von der Desinfektionsmittelquelle (607), Basenquelle (608), Wasserquelle (605) und/oder Salzlösungsquelle (606) zum ersten Sorptionsmittelmoduleinlass (643); und
mindestens eine zweite Pumpe (631, 632) zum Pumpen von Fluid von der Desinfektionsmittelquelle (607), Basenquelle (608), Wasserquelle (605) und/oder Salzlösungsquelle (606) zur Verzweigungsstelle (665);
wobei sich die mindestens zweite Pumpe (631, 632) zum Pumpen von Fluid von der Desinfektionsmittelquelle (607), Basenquelle (608), Wasserquelle (605) und/oder Salzlösungsquelle (606) zu dem zweiten Sorptionsmittelmoduleinlass vorzugsweise in der zweiten Einlassleitung befindet; und
das Sorptionsmittelladegerät ferner eine Steuerung zum Steuern der ersten Pumpe und der zweiten Pumpe (631, 632) umfasst.

5. Sorptionsmittelladegerät nach Anspruch 4, wobei die Steuerung dazu konfiguriert ist, die erste Pumpe (609, 610) so zu steuern, dass diese Fluid von der Salzlösungsquelle (606) zu dem ersten Sorptionsmittelmoduleinlass (643) pumpt; und
die zweite Pumpe (631, 632) so zu steuern, dass diese gleichzeitig Fluid von der Basenquelle (608) zu dem zweiten Sorptionsmittelmoduleinlass pumpt.

6. Sorptionsmittelladegerät nach Anspruch 2, ferner umfassend mindestens eine Modul-Bypassleitung (628, 642), wobei die Modul-Bypassleitung die Fluidverbindung herstellt entweder:
zwischen der ersten Einlassleitung und der ersten Abflussleitung; oder
der zweiten Einlassleitung und der zweiten Abflussleitung; und/oder
wobei der erste Sorptionsmittelmoduleinlass in Fluidverbindung mit dem ersten Sorptionsmittelmodulauslass gebracht werden kann und/oder der zweite Sorptionsmittelmoduleinlass in Fluidverbindung mit dem zweiten Sorptionsmittelmodulauslass gebracht werden kann.

7. Sorptionsmittelladegerät nach einem der Ansprüche 1 bis 8, ferner umfassend einen pH-Sensor, der dem statischen Mischer nachgelagert ist.

8. Verfahren, umfassend die folgenden Schritte:
Pumpen einer Salzlösung durch ein erstes Sorptionsmittelmodul (603), das Zirkoniumphosphat enthält, in ein Sorptionsmittelladegerät nach Anspruch 1;
Pumpen der sich daraus ergebenden Lösung zu einem statischen Mischer (638) oder einem gemeinsamen Tank; und
gleichzeitiges Pumpen einer basischen Lösung zu dem statischen Mischer oder gemeinsamen Tank.

9. Verfahren nach Anspruch 8, wobei der Schritt des Pumpens der basischen Lösung zu dem statischen Mischer (638) oder einem gemeinsamen Tank den Schritt des Pumpens der basischen Lösung durch ein zweites Sorptionsmittelmodul (604) umfasst, wobei das zweite Sorptionsmittelmodul Zirkoniumoxid enthält; und/oder
wobei der Schritt des Pumpens der basischen Lösung zu dem statischen Mischer den Schritt des Pumpens der basischen Lösung durch eine Modul-Bypassleitung (628, 642) umfasst; und/oder
ferner umfassend den Schritt des Pumpens von Fluid von dem statischen Mischer (638) zu einer Ableitung (653); und/oder
ferner umfassend den Schritt des Erfassens einer Leitfähigkeit oder eines pH-Werts in einem Abfluss des Sorptionsmittelmoduls (603), das Zirkoniumphosphat enthält, mit einem Leitfähigkeitssensor (619, 639), einem pH-Sensor oder Kombinationen davon; und
wobei der Schritt des gleichzeitigen Pumpens einer basischen Lösung zu dem statischen Mischer (638) ausgeführt wird, wenn der Leitfähigkeitssensor (619, 639) in dem Abfluss des Sorptionsmittelmoduls (603), das Zirkoniumphosphat enthält, eine Zunahme der Leitfähigkeit erfasst, der pH-Sensor eine Abnahme des pH-Werts erfasst oder Kombinationen davon.

10. Verfahren, umfassend die folgenden Schritte:
Pumpen einer basischen Lösung durch ein erstes Sorptionsmittelmodul (604), das Zirkoniumoxid enthält, in ein Sorptionsmittelladegerät nach Anspruch 1;
Pumpen einer sich daraus ergebenden Lösung zu einem statischen Mischer (638) oder einem gemeinsamen Tank; und
gleichzeitiges Pumpen einer Salzlösung zu dem statischen Mischer (638) oder einem gemeinsamen Tank.

11. Verfahren nach Anspruch 10, wobei der Schritt des Pumpens der Salzlösung zu dem statischen Mischer (638) oder dem gemeinsamen Tank den Schritt des Pumpens der Salzlösung durch ein zweites Sorptionsmittelmodul (603) umfasst, wobei das zweite Sorptionsmittelmodul Zirkoniumphosphat enthält; und/oder
wobei der Schritt des Pumpens der Salzlösung zu dem statischen Mischer (638) oder dem gemeinsamen Tank den Schritt des Pumpens der Salzlösung durch eine Modul-Bypassleitung (628, 642) umfasst.

12. Verfahren nach Anspruch 10, ferner umfassend den Schritt des Erfassens einer Leitfähigkeit oder eines pH-Werts in einem Abfluss des Sorptionsmittelmoduls (604), das Zirkoniumoxid enthält, mit einem Leitfähigkeitssensor (619, 639), einem pH-Sensor oder Kombinationen davon; und
wobei der Schritt des gleichzeitigen Pumpens einer Salzlösung zu dem statischen Mischer (638) ausgeführt wird, wenn der Leitfähigkeitssensor (619, 639) in dem Abfluss des Sorptionsmittelmoduls (604), das Zirkoniumoxid enthält, eine Zunahme der Leitfähigkeit erfasst, der pH-Sensor eine Zunahme des pH-Werts erfasst oder Kombinationen davon.

13. Verfahren nach einem der Ansprüche 8 bis 12, ferner umfassend:
Bestimmen eines pH-Wertes in dem Fluid, das dem statischen Mischer (638) nachgelagert ist, mit einem Sensor; und
Pumpen des Fluids aus dem statischen Mischer (638) zu einer Ableitung (653), wenn der pH-Wert des Fluids zwischen 5 und 9 liegt.

## Revendications

1. Dispositif de recharge de sorbant, comprenant :
au moins un premier compartiment de réception pour un premier module de sorbant (604) ;
le premier compartiment de réception ayant une première entrée de module de sorbant (643) et une première sortie de module de sorbant (644) ;
une première conduite d'entrée raccordée fluidiquement à l'entrée du premier module de sorbant (643) ;
une première conduite d'effluent (645) raccordée fluidiquement à la sortie du premier module de sorbant ;
une source de désinfectant (607), une source de base (608), une source d'eau (605) et une source de saumure (606) ; l'une quelconque parmi la source de désinfectant (607), la source de base (608), la source d'eau (605), la source de saumure (606) et leurs combinaisons étant raccordée fluidiquement à la première conduite d'entrée ; et la source de désinfectant (607) et/ou la source de base (608) et/ou la source d'eau (605) et/ou la source de saumure (606) étant raccordée fluidiquement à la première conduite d'effluent au niveau d'une jonction (665) ; et
un mélangeur statique (638) au niveau ou en aval de la jonction (665) ;
a) la source d'eau (605), la source de désinfectant (607) et la source de saumure (606) étant raccordées fluidiquement à la première conduite d'entrée ; et au moins la source d'eau (605) et la source de base (608) étant raccordées fluidiquement à la première conduite d'effluent ; ou
b) la source d'eau (605), la source de désinfectant (607) et la source de base (608) étant raccordées fluidiquement à la première conduite d'entrée ; et au moins la source d'eau (605) et la source de saumure (606) étant raccordées fluidiquement à la première conduite d'effluent (645).

2. Dispositif de recharge de sorbant selon la revendication 1, le dispositif de recharge de sorbant comprenant un second compartiment de réception pour un second module de sorbant réutilisable (603) ;
le second compartiment de réception comprenant une seconde entrée de module de sorbant et une seconde sortie de module de sorbant ;
une seconde conduite d'entrée raccordée fluidiquement à l'entrée du second module de sorbant ;
une seconde conduite d'effluent (630) raccordée fluidiquement à la sortie du second module de sorbant ;
la source de désinfectant (607) et/ou la source de base (608) et/ou la source d'eau (605) et/ou la source de saumure (606) étant raccordées fluidiquement à la seconde conduite d'entrée ; et la seconde conduite d'effluent (630) étant raccordée fluidiquement à la jonction (665).

3. Dispositif de recharge de sorbant selon la revendication 1 ou la revendication 2, comprenant en outre un capteur de conductivité (619, 639) positionné dans la première conduite d'effluent ; comprenant en outre de préférence un capteur de conductivité (619, 639) positionné dans chacune de la première conduite d'effluent (645) et de la seconde conduite d'effluent (630).

4. Dispositif de recharge de sorbant selon l'une quelconque des revendications précédentes, comprenant en outre au moins une première pompe (609, 610) dans la première conduite d'entrée pour pomper du fluide depuis la source de désinfectant (607), la source de base (608), la source d'eau (605) et/ou la source de saumure (606) jusqu'à l'entrée du premier module de sorbant (643) ; et
au moins une seconde pompe (631, 632) pour pomper du fluide depuis la source de désinfectant (607), la source de base (608), la source d'eau (605) et/ou la source de saumure (606) jusqu'à la jonction (665) ;
de préférence l'au moins seconde pompe (631, 632) se trouvant dans la seconde conduite d'entrée pour pomper du fluide depuis la source de désinfectant (607), la source de base (608), la source d'eau (605) et/ou la source de saumure (606) jusqu'à l'entrée du second module de sorbant ; et
le dispositif de recharge de sorbant comprenant en outre un dispositif de commande pour commander la première pompe et la seconde pompe (631, 632).

5. Dispositif de recharge de sorbant selon la revendication 4, le dispositif de commande étant conçu pour commander la première pompe (609, 610) pour pomper du fluide depuis la source de saumure (606) jusqu'à l'entrée du premier module de sorbant (643) ; et
pour commander la seconde pompe (631, 632) pour pomper du fluide depuis la source de base (608) jusqu'à l'entrée du second module de sorbant simultanément.

6. Dispositif de recharge de sorbant selon la revendication 2, comprenant en outre au moins une conduite de dérivation de module (628, 642), la conduite de dérivation de module raccordant fluidiquement soit : la première conduite d'entrée à la première conduite d'effluent ;
ou la seconde conduite d'entrée à la seconde conduite d'effluent ;
et/ou l'entrée du premier module de sorbant pouvant être raccordée fluidiquement à la sortie du premier module de sorbant et/ou l'entrée du second module de sorbant pouvant être raccordée fluidiquement à la sortie du second module de sorbant.

7. Dispositif de recharge de sorbant selon l'une quelconque des revendications 1 à 8, comprenant en outre un capteur de pH en aval du mélangeur statique.

8. Procédé comprenant les étapes consistant à :
pomper une solution de saumure à travers un premier module de sorbant (603) contenant du phosphate de zirconium dans un dispositif de recharge de sorbant selon la revendication 1 ;
pomper la solution obtenue jusqu'à un mélangeur statique (638) ou jusqu'à un réservoir commun ; et
pomper simultanément une solution de base jusqu'au mélangeur statique ou jusqu'au réservoir commun.

9. Procédé selon la revendication 8, l'étape de pompage de la solution de base jusqu'au mélangeur statique (638) ou jusqu'au réservoir commun comprenant l'étape de pompage de la solution de base à travers un second module de sorbant (604), le second module de sorbant contenant de l'oxyde de zirconium ;
et/ou l'étape de pompage de la solution de base jusqu'au mélangeur statique comprenant l'étape de pompage de la solution de base à travers une conduite de dérivation de module (628, 642) ;
et/ou comprenant en outre l'étape de pompage de fluide du mélangeur statique (638) jusqu'à un drain (653) ;
et/ou comprenant en outre l'étape de détection d'une conductivité ou d'un pH dans un effluent du module de sorbant (603) contenant du phosphate de zirconium avec un capteur de conductivité (619, 639), un capteur de pH, ou des combinaisons de ceux-ci ; et l'étape de pompage simultanée d'une solution de base jusqu'au mélangeur statique (638) étant effectuée lorsque le capteur de conductivité (619, 639) détecte une augmentation de conductivité, le capteur de pH détectant une diminution du pH, ou des combinaisons de ceux-ci, dans l'effluent du module de sorbant (603) contenant du phosphate de zirconium.

10. Procédé comprenant les étapes consistant à :
pomper une solution de base à travers un premier module de sorbant (604) contenant de l'oxyde de zirconium dans un dispositif de recharge de sorbant selon la revendication 1 ;
pomper une solution résultante jusqu'à un mélangeur statique (638) ou jusqu'à un réservoir commun ; et
pomper simultanément une solution de saumure jusqu'au mélangeur statique (638) ou jusqu'au réservoir commun.

11. Procédé selon la revendication 10, l'étape de pompage de la solution de saumure jusqu'au mélangeur statique (638) ou jusqu'au réservoir commun comprenant l'étape de pompage de la solution de saumure à travers un second module de sorbant (603), le second module de sorbant contenant du phosphate de zirconium ; et/ou
l'étape de pompage de la solution de saumure jusqu'au mélangeur statique (638) ou jusqu'au réservoir commun comprenant l'étape de pompage de la solution de saumure à travers une conduite de dérivation de module (628, 642).

12. Procédé selon la revendication 10, comprenant en outre l'étape de détection d'une conductivité ou d'un pH dans un effluent du module de sorbant (604) contenant de l'oxyde de zirconium avec un capteur de conductivité (619, 639), un capteur de pH, ou des combinaisons de ceux-ci ; et
l'étape de pompage de manière simultanée d'une solution de saumure jusqu'au mélangeur statique (638) étant effectuée lorsque le capteur de conductivité (619, 639) détecte une augmentation de conductivité, le capteur de pH détectant une augmentation du pH, ou des combinaisons de ceux-ci, dans l'effluent du module de sorbant (604) contenant de l'oxyde de zirconium.

13. Procédé selon l'une des revendications 8 à 12, comprenant en outre :
la détermination d'un pH dans le fluide en aval du mélangeur statique (638) avec un capteur ; et
le pompage du fluide depuis le mélangeur statique (638) jusqu'à un drain (653) si le pH du fluide est compris entre 5 et 9.
